(19) Europäisches Patentamt
European Patent Office
Office européen des brevets

(11) EP 3 332 793 B1

(12) **FASCICULE DE BREVET EUROPEEN**

(45) Date de publication et mention de la délivrance du brevet:
**18.03.2020 Bulletin 2020/12**

(21) Numéro de dépôt: **17203260.9**

(22) Date de dépôt: **20.10.2015**

(51) Int Cl.:
*A61K 31/197* (2006.01) *A61K 31/4415* (2006.01)
*A61K 36/28* (2006.01) *A61K 36/45* (2006.01)
*A61K 36/67* (2006.01) *A61K 31/4188* (2006.01)
*A61K 31/4525* (2006.01) *A61K 31/455* (2006.01)
*A61K 31/51* (2006.01) *A61K 31/525* (2006.01)
*A61K 31/555* (2006.01) *A61K 31/593* (2006.01)
*A61K 31/714* (2006.01) *A61K 33/30* (2006.01)
*A23L 33/105* (2016.01)

(54) **COMPOSITION COMPRENANT UN MELANGE D'EXTRAITS VEGETAUX OU UN MELANGE DE MOLECULES CONTENUES DANS CES VEGETAUX ET UTILISATION POUR AGIR SUR LE METABOLISME GLUCIDIQUE ET/OU LIPIDIQUE**

ZUSAMMENSETZUNG, DIE EINE MISCHUNG AUS PFLANZENEXTRAKTEN ODER MOLEKÜLEN, DIE IN DIESEN PFLANZEN ENTHALTEN SIND, UMFASST, UND EINSATZ ZUR EINWIRKUNG AUF DEN ZUCKER- UND/ODER FETTSTOFFWECHSEL

COMPOSITION COMPRISING A MIXTURE OF PLANT EXTRACTS OR A MIXTURE OF MOLECULES CONTAINED IN SAID PLANTS AND USE TO ACT ON THE CARBOHYDRATE AND/OR LIPID METABOLISM

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**
Etats d'extension désignés:
**BA ME**
Etats de validation désignés:
**MA**

(30) Priorité: **20.10.2014 FR 1460064**

(43) Date de publication de la demande:
**13.06.2018 Bulletin 2018/24**

(62) Numéro(s) de document de la (des) demande(s) initiale(s) en application de l'article 76 CBE:
**15801895.2 / 3 209 315**

(73) Titulaires:
- **Valbiotis**
  **17180 Perigny (FR)**
- **Université Clermont Auvergne**
  **63000 Clermont-Ferrand (FR)**
- **Universite De La Rochelle**
  **17071 La Rochelle Cedex 9 (FR)**
- **CNRS**
  **75794 Paris Cedex 16 (FR)**

(72) Inventeurs:
- **PELTIER, Sébastien**
  **17450 FOURAS (FR)**
- **SIRVENT, Pascal**
  **63122 CEYRAT (FR)**
- **MAUGARD, Thierry**
  **17220 LA JARNE (FR)**

(74) Mandataire: **Aquinov**
**Allée de la Forestière**
**33750 Beychac et Caillau (FR)**

(56) Documents cités:
**FR-A1- 2 908 309** **US-A1- 2011 288 125**
**US-A1- 2013 266 638**

- **DATABASE WPI Week 201414 Thomson Scientific, London, GB; AN 2013-V44914 XP002742306, & CN 103 271 194 A (WANG Y) 4 septembre 2013 (2013-09-04)**
- **DATABASE WPI Week 201433 Thomson Scientific, London, GB; AN 2014-J46645 XP002742307, & CN 103 652 799 A (FUYANG JIUZHEN FOOD CO LTD) 26 mars 2014 (2014-03-26)**
- **DATABASE WPI Week 201444 Thomson Scientific, London, GB; AN 2014-L88165 XP002742308, & CN 103 750 439 A (MA'ANSHAN ANKANG FUNGUS IND CO LTD) 30 avril 2014 (2014-04-30)**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

- **DATABASE WPI Week 201438 Thomson Scientific, London, GB; AN 2014-L11875 XP002742309, & CN 103 719 269 A (MA'ANSHAN ANKANG FUNGUS IND CO LTD) 16 avril 2014 (2014-04-16)**
- **CYRIL JANIN: "Recette : artichauts avec trois sauces aux myrtilles sauvages", INTERNET CITATION, 19 octobre 2014 (2014-10-19), pages 1-5, XP002753164, Extrait de l'Internet: URL:http://www.keldelice.com/cuisine-du-terroir/recettes/artichauts-avec-trois-sauces-aux-myrtilles-sauvages [extrait le 2016-01-19]**
- **EDDOUKS MOHAMED ET AL: "Antidiabetic plants improving insulin sensitivity", JOURNAL OF PHARMACY AND PHARMACOLOGY, vol. 66, no. 9, septembre 2014 (2014-09), pages 1197-1214, XP002742310, ISSN: 0022-3573**
- **"Chrysanthellum", WikiPhyto , 27 mars 2013 (2013-03-27), page 5PP, XP002742311, Extrait de l'Internet: URL:http://www.wikiphyto.org/wiki/Chrysanthellum [extrait le 2015-07-14]**
- **WikiPhyto: "Poivrier commun", , 17 août 2013 (2013-08-17), page 4PP, XP002742312, Extrait de l'Internet: URL:http://www.wikiphyto.org/wiki/Poivrier_commun [extrait le 2015-07-14]**


Remarques:

Le dossier contient des informations techniques présentées postérieurement au dépôt de la demande et ne figurant pas dans le présent fascicule.

## Description

La présente invention concerne la prévention et le traitement des dérèglements du métabolisme glucidique et/ou lipidique chez l'Homme ou l'animal.

Le diabète de type 2, la forme la plus fréquente de diabète, est une maladie métabolique chronique, progressive. Elle est caractérisée par une hyperglycémie chronique, c'est-à-dire une concentration en sucre dans le sang anormalement élevée, et une intolérance aux glucides. La principale cause de cet état hyperglycémique chronique est une résistance à l'insuline ainsi que sa sécrétion inadéquate en réponse à un état métabolique donné, mais d'autres facteurs peuvent intervenir (Ismail-Beigi F N Engl J Med 2012; 366:1319-27). Pour lutter contre le diabète de type 2, il convient principalement de réduire les niveaux de glycémie, de diminuer l'hémoglobine glyquée ($HbA_1c$) à un taux inférieur ou égal à 6.5%, et d'améliorer la sensibilité à l'insuline.

Le traitement du diabète de type 2 consiste tout d'abord en une modification du mode de vie ou mesures hygiéno-diététiques (MHD : alimentation, tabac, activités physiques et sportives). Si les MHD ne suffisent pas, il convient alors d'avoir recours à des agents thérapeutiques antidiabétiques, généralement de la metformine. Or, la metformine présente de nombreuses contre-indications médicales, telles que l'insuffisance rénale chronique, l'acidose, l'hypoxie, la déshydratation, etc. Il existe donc un paradoxe important pour la metformine puisque sa prescription n'est pas possible chez les patients diabétiques de type 2 qui présentent une insuffisance rénale, or l'insuffisance rénale est une des conséquences fréquentes du diabète de type 2. D'autres agents thérapeutiques ont été développés comme les inhibiteurs de la dipeptidyl peptidase-IV (DPP-IV), des analogues au glucagon-like peptide-1 (GLP-1), des thiazolidinediones (TZDs), des sulfonylurées, des inhibiteurs des glycosidases (acarbose, miglitol, voglibose), ou encore des inhibiteurs du sodium glucose co-transporter-2 (SGLT2). Toutefois, la bonne combinaison thérapeutique est complexe car elle nécessite la prise en considération d'un nombre important de facteurs comme les contre-indications et les effets indésirables, qui affectent la qualité de vie des patients et donc leur adhérence au traitement médical. De plus, certaines combinaisons thérapeutiques augmentent la mortalité toutes causes comme l'association de la metformine aux sulfonylurées (Prescrire Int 2015; 24:130-5).

Les MHD n'étant que très rarement suivies par les patients, cela nécessite très rapidement d'instaurer un traitement thérapeutique avec tous les effets indésirables et contre-indications liées à ces molécules, et il n'existe pas aujourd'hui de solution adaptée à la prise en charge des patients diabétiques de type 2 entre les MHD et l'instauration d'un traitement thérapeutique.

Par ailleurs, les patients diabétiques de type 2 ont un risque élevé de morbi-mortalité cardiovasculaire. Il est donc également nécessaire de prendre en charge les facteurs de risque cardiovasculaire traditionnels comme notamment le contrôle des lipides circulants, du poids et de la pression artérielle. Cette nécessité induit actuellement la prise de plusieurs médicaments de différentes classes thérapeutiques simultanément. Or, la combinaison de drogues peut parfois engendrer des réactions secondaires graves comme par exemple, l'administration simultanée de fibrates et de statines qui augmente le risque de myopathie (Denke MA J Manag Care Pharm 2003; 9:17-9).

Il existe donc un besoin réel de produits pouvant être utilisés à la fois lors de l'instauration sourde des pathologies cardio-métaboliques, caractérisée par une élévation de certains facteurs de risque (troubles glucidiques, troubles lipidiques, surpoids, inflammation, stress oxydant, hypertension artérielle), et lors du déclenchement de ces pathologies, diabète de type 2 notamment.

Par ailleurs, il existe un besoin urgent de solutions de prévention et de médicaments dont le mécanisme d'action « multicibles » présente des avantages en termes de compliance, de tolérance et d'efficacité. De tels produits permettraient de diminuer le risque global de maladies cardio-métaboliques et de prévenir et traiter chaque dysfonctionnement et/ou ses conséquences pris indépendamment.

L'objectif de l'invention est de répondre à ces différents besoins en proposant des compositions capables d'agir de manière simultanée sur plusieurs dysfonctionnements glucidiques et lipidiques, qui représentent à la fois un moyen de prévention et un moyen thérapeutique avantageux pour prévenir et traiter les maladies cardio-métaboliques et leurs complications.

Pour répondre à son objectif, l'invention vise des compositions comprenant au moins un mélange de molécules obtenues au moins à partir de :

- *Chrysanthellum indicum,* et
- *Cynara scolymus,* et
- *Vaccinium myrtillus,* et
- *Olea europaea*

ledit mélange de molécules comprenant également de la pipérine.

Il s'agit d'un mélange synergique, les molécules présentes dans le mélange agissant en synergie.

Des extraits de *Chrysanthellum indicum,* des extraits de *Cynara scolymus,* des extraits de *Vaccinium myrtillus*

*et la pipérine* ont déjà été décrits et certains ont été utilisés dans des produits de nutrition, mais de façon inattendue la combinaison d'au moins un extrait de *Chrysanthellum indicum,* avec au moins un extrait de *Cynara scolymu,* un extrait de *Vaccinium myrtillus et de la pipérine* conduit à des résultats surprenants à la fois sur le métabolisme glucidique et sur le métabolisme lipidique chez l'Homme ou l'animal.

Pour répondre à son objectif, l'invention vise aussi des compositions constituées par au moins de la pipérine et des molécules spécifiques particulières contenues dans *Chrysanthellum indicum, Cynara scolymus, Vaccinium myrtillus* et Olea europaea (ces molécules étant naturelles et/ou de synthèse), en particulier une composition comprenant un mélange d'au moins cinq molécules, au moins une molécule étant de la pipérine, au moins une molécule étant choisie parmi l'oleuropéine et l'hydroxytyrosol, au moins une molécule étant choisie parmi l'apigenine-7-O-glucuronide, la chrysanthelline A, la chrysanthelline B, l'acide caféique, la lutéoline, la maritimétine, l'ériodictyol, l'isookanine, l'apigénine, la lutéoline-7-O-glucoside, la maritiméine, la maréine, l'ériodictyol-7-O-glucoside, la flavomaréine, l'apigénine-8-C-α-L-arabinoside-6-C-β-D-glucoside (shaftoside), l'apigénine-6,8-C-di-β-D-glucopyranoside (vicénine-2), et au moins une molécule étant choisie parmi un acide dicaféoylquinique, un acide sulfo-monocaféoylquinique, la lutéoline, la lutéoline-7-0-glucoside, la lutéoline-7-O-glucuronide, l'apigenine-7-O-glucoside, la cynaropicrine, et au moins une molécule étant choisie parmi un acide monocaféoylquinique, la delphinidine-3-galactoside, la delphinidine-3-glucoside, la cyanidine-3-galactoside, la delphinidine-3-arabinoside, la cyanidine-3-glucoside, la petunidine-3-galactoside, la cyanidine-3-arabinoside, la petunidine-3-glucoside, la péonidine-3-galactoside, la petunidine-3-arabinoside, la péonidine-3-glucoside, la malvidine-3-galactoside, la malvidine-3-glucoside, la malvidine-3-arabinoside, ou leurs analogues.

Dans la présente demande, le singulier ou le pluriel seront indifféremment utilisés pour désigner les compositions selon l'invention.

Avantageusement, les compositions selon l'invention empêchent l'instauration d'une hyperglycémie chronique et diminuent la glycémie, diminuent l'hémoglobine glyquée, permettent d'améliorer la tolérance aux glucides ingérés, améliorent la sensibilité à l'insuline, mais elles sont également capables d'agir sur d'autres facteurs de risque cardiovasculaire comme la dyslipidémie, le surpoids et l'obésité, et la tension artérielle. En outre, elles ne présentent pas ou peu d'effets secondaires en regard de ceux observés avec les traitements existants et ceux en cours de développement.

L'invention vise donc également l'utilisation des compositions comme produits nutritionnels ou produits de santé, notamment comme médicaments, en particulier pour prévenir et/ou lutter contre les dérèglements du métabolisme glucidique et lipidique chez l'Homme ou l'animal.

L'invention est à présent décrite en détail en regard des figures annexées qui représentent :

- Figure 1 : les résultats pour quatre compositions d'un test oral de sensibilité à l'insuline correspondants aux résultats du tableau 9 (point II.1).
- Figure 2 : les résultats démontrant l'effet de synergie apporté par une composition selon l'invention, ces résultats correspondants aux résultats du tableau 13 (point II.1) pour la glycémie à jeun.
- Figure 3 : les résultats démontrant l'effet de synergie apporté par une composition selon l'invention, ces résultats correspondants aux résultats du tableau 13 (point II.1) pour la tolérance aux glucides.
- Figure 4 : les résultats démontrant l'effet de synergie apporté par une composition selon l'invention, ces résultats correspondants aux résultats du tableau 14 (point II.1) pour la glycémie à jeun.
- Figure 5 : les résultats démontrant l'effet de synergie apporté par une composition selon l'invention, ces résultats correspondants aux résultats du tableau 14 (point II.1) pour la tolérance aux glucides.
- Figure 6 : les résultats démontrant l'effet de synergie apporté par une composition selon l'invention, ces résultats correspondants aux résultats du tableau 14 (point II.1) pour la sensibilité à l'insuline.
- Figure 7 : les résultats pour l'effet d'une composition selon l'invention et la metformine, sur la sensibilité à l'insuline, ces résultats correspondants aux résultats du tableau 15 (point II.2).
- Figure 8 : les résultats pour l'effet d'une composition selon l'invention sur l'évolution de la masse corporelle, ces résultats correspondants aux résultats du tableau 15 (point II.2).
- Figure 9 : les résultats pour l'effet d'une composition selon l'invention sur l'évolution de la glycémie à jeun, ces résultats correspondants aux résultats du tableau 15 (point II.2).
- Figure 10 : les résultats d'un western blot montrant une augmentation de la quantité d'AMPK au niveau du foie de souris avec une composition selon l'invention (point II.2).
- Figure 11 : les résultats de tests montrant l'absence d'effet hypoglyécmiant en aigue avec une composition selon l'invention (point II.3).
- Figure 12 : les résultats de tests montrant l'effet d'une composition selon l'invention sur l'évolution de la prise de masse corporelle (point II.4).

L'invention a pour objet une composition comprenant au moins un mélange de molécules obtenues au moins à partir de :

- *Chrysanthellum indicum,* et
- *Cynara scolymus,* et
- *Vaccinium myrtillus,* et
- *Olea europea*

ledit mélange de molécules comprenant également de la pipérine.

L'invention vise donc une composition comprenant au moins de la pipérine, au moins une molécule issue de *Chrysanthellum indicum*, au moins une molécule issue de *Cynara scolymus* au moins une molécule issue de *Vaccinium myrtillus*, et au moins une molécule issue de l'*Olea europea.*

En plus de *Chrysanthellum indicum,* de *Cynara scolymus,* de *Vaccinium myrtillus,* d'*Olea europaea* et de la pipérine, la composition selon l'invention peut également contenir d'autres composés.

Selon un premier mode de réalisation, la composition selon l'invention comprend au moins :

- un extrait de *Chrysanthellum indicum,* et
- un extrait de *Cynara scolymus,* et
- un extrait de *Vaccinium myrtillus, et*
- un extrait d'*Olea europaea, et*
- de la pipérine synthétique et/ou un extrait de *Piper* contenant de la pipérine.

Par extrait de plante « X » ou de matière première végétale « X » au sens de l'invention, on entend au moins une molécule, préférentiellement un ensemble de molécules obtenu à partir de la plante « X » par tout procédé adapté. On peut en particulier citer les extraits aqueux (obtenus à l'aide d'un solvant aqueux), alcooliques (obtenus à l'aide d'un solvant alcoolique) ou utilisant un solvant organique, ou à l'aide d'un corps gras naturel ou un mélange de corps gras naturels, notamment une huile végétale ou un mélange d'huiles végétales. Par solvant aqueux on entend tout solvant constitué totalement ou pour part d'eau. On peut citer ainsi l'eau elle-même, les solvants hydro-alcooliques en toute proportion ou encore les solvants constitués d'eau et d'un composé comme la glycérine ou le propylène glycol en toute proportion. Parmi les solvants alcooliques on peut citer notamment l'éthanol.

Par plante ou matière première végétale au sens de l'invention, on entend la plante entière ou la partie de plante, incluant les cultures de cellules, n'ayant pas encore subi de traitement spécifique et destinée à entrer dans la fabrication d'une préparation de plante.

Les extraits de plantes peuvent être obtenus par tout procédé adapté, par exemple par un procédé comprenant les étapes suivantes :

- extraction solide / liquide
- séparation / pressage
- filtration
- évaporation
- séchage
- éventuellement incorporation d'additifs
- homogénéisation
- conditionnement.

L'extrait de *Chrysanthellum indicum* est de façon préférée un extrait de plante entière ou des parties aériennes.

Il peut s'agir en particulier d'un extrait hydroalcoolique ou aqueux ou $CO_2$ subcritique ou $H_20$ subcritique ou associé à un traitement thermique réalisé par chauffage classique ou sous fréquence micro-ondes ou sous ultrasons.

Le ratio plante / extrait est préférentiellement compris entre 1/1 à 100/1, en particulier entre 1/1 et 25/1.

La composition selon l'invention, lorsqu'elle est destinée à l'Homme, comprend préférentiellement une quantité d'extrait de *Chrysanthellum indicum* permettant l'administration d'au moins 0,00001g, en particulier entre 0,00001g et 0,60g, d'extrait de *Chrysanthellum indicum* par kg de poids corporel de la personne à laquelle la composition est administrée et par jour.

De façon préférée, l'extrait de *Chrysanthellum indicum* comprend au moins une molécule choisie parmi l'apigenine-7-O-glucuronide, la chrysanthelline A, la chrysanthelline B, l'acide caféique, la lutéoline, la maritimétine, l'ériodictyol, l'isookanine, l'apigénine, la lutéoline-7-O-glucoside, la maritiméine, la maréine, l'ériodictyol-7-O-glucoside, la flavomaréine, l'apigénine-8-C-α-L-arabinoside-6-C-β-D-glucoside (shaftoside), l'apigénine-6,8-C-di-β-D-glucopyranoside (vicénine-2), ou leurs analogues. Préférentiellement l'extrait comprend au moins l'apigenine-7-O-glucuronide.

Par analogues au sens de la présente invention on entend tous composés ayant une structure chimique similaire à un autre composé, mais différent de celui-ci par un certain composant. Il peut différer de un ou plusieurs atomes, groupes fonctionnels, sous-structures, qui sont remplacés par d'autres atomes, groupes fonctionnels ou sous-structures.

On peut citer par exemple les analogues de l'apigenine-7-O-glucuronide comme l'apigénine-7-apioglucoside, l'apigénine-8-C-glucoside (vitexine), l'apigénine-6-C-glucoside (isovitexine), l'apigénine-7-O-néohespéridoside, l'apigénine-7-glucoside, l'apigénine-7-apioglucoside.

L'extrait de *Cynara scolymus* est de façon préférée un extrait de feuilles ou de racines.

Il peut s'agir en particulier d'un extrait hydroalcoolique ou aqueux ou $CO_2$ subcritique ou $H_20$ subcritique ou associé à un traitement thermique réalisé par chauffage classique ou sous fréquence micro-ondes ou sous ultrasons.

Le ratio plante / extrait est préférentiellement compris entre 1/1 à 100/1, en particulier entre 1/1 et 30/1.

La composition selon l'invention, lorsqu'elle est destinée à l'Homme, comprend préférentiellement une quantité d'extrait de *Cynara scolymus* permettant l'administration d'au moins 0,00001g, en particulier entre 0,00001g et 0,60g, d'extrait de *Cynara scolymus* par kg de poids corporel de la personne à laquelle la composition est administrée et par jour. De façon préférée, l'extrait de *Cynara scolymus* comprend au moins une molécule choisie parmi un acide dicaféoylquinique, un acide sulfo-monocaféoylquinique, la lutéoline, la lutéoline-7-0-glucoside, la lutéoline-7-O-glucuronide, l'apigenine-7-O-glucoside, la cynaropicrine, ou leurs analogues. Préférentiellement, l'extrait comprend au moins un acide dicaféoylquinique.

L'extrait de *Vaccinium myrtillus* est préférentiellement un extrait de fruits ou de feuilles.

Il peut s'agir en particulier d'un extrait hydroalcoolique ou aqueux ou $CO_2$ subcritique ou $H_20$ subcritique ou associé à un traitement thermique réalisé par chauffage classique ou sous fréquence micro-ondes ou sous ultrasons.

Le ratio plante / extrait est préférentiellement compris entre 1/1 à 200/1, en particulier entre 1/1 et 60/1.

La composition selon l'invention, lorsqu'elle est destinée à l'Homme, comprend préférentiellement une quantité d'extrait de *Vaccinium myrtillus* permettant l'administration d'au moins 0,00001g, en particulier entre 0,00001g et 0,60g, d'extrait de *Vaccinium myrtillus* par kg de poids corporel de la personne à laquelle la composition est administrée et par jour.

De façon préférée, l'extrait de *Vaccinium myrtillus* comprend au moins une molécule choisie parmi un acide monocaféoylquinique, la delphinidine-3-galactoside, la delphinidine-3-glucoside, la cyanidine-3-galactoside, la delphinidine-3-arabinoside, la cyanidine-3-glucoside, la petunidine-3-galactoside, la cyanidine-3-arabinoside, la petunidine-3-glucoside, la péonidine-3-galactoside, la petunidine-3-arabinoside, la péonidine-3-glucoside, la malvidine-3-galactoside, la malvidine-3-glucoside, la malvidine-3-arabinoside, ou leurs analogues. Préférentiellement, l'extrait comprend au moins un acide monocaféoylquinique.

La pipérine présente dans la composition selon l'invention, peut être contenue dans un extrait de *Piper* ou bien être une pipérine synthétique.

La formule topologique de la pipérine est la suivante :

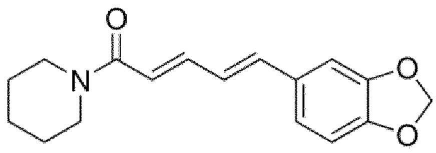

La composition selon l'invention, lorsqu'elle est destinée à l'Homme, comprend préférentiellement une quantité de pipérine permettant l'administration d'au moins 0,001mg, en particulier entre 0,001mg et 166 mg, de pipérine par kg de poids corporel de la personne à laquelle la composition est administrée et par jour.

Si la pipérine est contenue dans un extrait de *Piper,* le mélange de la composition selon l'invention comprend ledit extrait. L'extrait de *Piper* est préférentiellement un extrait de *Piper nigrum,* de *Piper aduncum* et/ou de *Piper longum.*

L'extrait de *Piper* est de façon préférée un extrait de fruits ou de feuilles.

Il peut s'agir en particulier d'un extrait hydroalcoolique ou aqueux ou $CO_2$ subcritique ou $H_20$ subcritique ou associé à un traitement thermique réalisé par chauffage classique ou sous fréquence micro-ondes ou sous ultrasons.

Le ratio plante / extrait est préférentiellement compris entre 1/1 et 10000/1, en particulier entre 1/1 et 200/1.

L'extrait comprend préférentiellement au moins 1% de pipérine en poids par rapport au poids total de l'extrait.

Aussi, le mélange de la composition selon l'invention comprend préférentiellement :

- au moins de la pipérine, et
- au moins une molécule choisie parmi l'apigenine-7-O-glucuronide, la chrysanthelline A, la chrysanthelline B, l'acide caféique, la lutéoline, la maritimétine, l'ériodictyol, l'isookanine, l'apigénine, la lutéoline-7-O-glucoside, la maritiméine, la maréine, l'ériodictyol-7-O-glucoside, la flavomaréine, l'apigénine-8-C-α-L-arabinoside-6-C-β-D-glucoside (shaftoside), l'apigénine-6,8-C-di-β-D-glucopyranoside (vicénine-2), ou leurs analogues, préférentiellement l'apigenine-7-O-glucuronide, et
- au moins une molécule choisie parmi un acide dicaféoylquinique, un acide sulfo-monocaféoylquinique, la lutéoline, la lutéoline-7-0-glucoside, la lutéoline-7-O-glucuronide, l'apigenine-7-O-glucoside, la cynaropicrine, ou leurs analogues, préférentiellement un acide dicaféoylquinique, et

- au moins une molécule choisie parmi un acide monocaféoylquinique, la delphinidine-3-galactoside, la delphinidine-3-glucoside, la cyanidine-3-galactoside, la delphinidine-3-arabinoside, la cyanidine-3-glucoside, la petunidine-3-galactoside, la cyanidine-3-arabinoside, la petunidine-3-glucoside, la péonidine-3-galactoside, la petunidine-3-arabinoside, la péonidine-3-glucoside, la malvidine-3-galactoside, la malvidine-3-glucoside, la malvidine-3-arabinoside, ou leurs analogues, préférentiellement au moins un acide monocaféoylquinique, et
- au moins une molécule choisie parmi l'oleuropéine et lh'ydroxytyrosol.

En plus des extraits de *Chrysanthellum indicum,* de *Cynara scolymus,* de *Vaccinium myrtillus,* d'Olea europaea et de la pipérine le mélange selon l'invention peut également contenir d'autres composés.

L'extrait d'*Olea europaea* est préférentiellement un extrait de feuilles ou de fruits.

Il peut s'agir en particulier d'un extrait hydroalcoolique ou aqueux ou $CO_2$ subcritique ou $H_20$ subcritique ou associé à un traitement thermique réalisé par chauffage classique ou sous fréquence micro-ondes ou sous ultrasons.

Le ratio plante / extrait est préférentiellement compris entre 1/1 à 200/1, en particulier entre 1/1 et 60/1.

La composition selon l'invention, lorsqu'elle est destinée à l'Homme, comprend préférentiellement une quantité d'extrait d'*Olea europaea* permettant l'administration d'au moins 0,00001g, en particulier entre 0,00001g et 0,60g, d'extrait d'*Olea europaea* par kg de poids corporel de la personne à laquelle la composition est administrée et par jour.

De façon préférée, l'extrait d'*Olea europaea* comprend au moins une molécule choisie parmi l'oleuropéine et l'hydroxytyrosol, ou leurs analogues.

Par mélange au sens de l'invention on entend l'association de substances (extraits et/ou molécules) sous forme solide, liquide ou gazeuse qui peuvent interagir ou non chimiquement. Le mélange d'extraits selon l'invention est obtenu par tout procédé connu de l'homme du métier. Il peut être obtenu par simple mélange des constituants. Préférentiellement, le ratio extrait de *Chrysanthellum indicum* / extrait de *Cynara scolymus* / extrait de *Vaccinium myrtillus* / pipérine dans le mélange est compris entre 0,01/0,01/0,01/0,0001 et 10/10/10/10.

Selon une variante, en plus du mélange constitué de plusieurs extraits de plantes ou à la place du mélange constitué par plusieurs extraits de plantes, le mélange de molécules de la composition selon l'invention peut comprendre au moins :

- un extrait unique obtenu à partir d'un mélange d'au moins deux plantes choisies parmi *Chrysanthellum indicum, Cynara scolymus, Olea europaea, Vaccinium myrtillus* et *Piper* et éventuellement,
- un extrait de *Chrysanthellum indicum* si le mélange de plantes de l'extrait unique ne comprend pas *Chrysanthellum indicum,*
- un extrait de *Cynara scolymus* si le mélange de plantes de l'extrait unique ne comprend pas *Cynara scolymus,*
- un extrait de *Vaccinium myrtillus* si le mélange de plantes de l'extrait unique ne comprend pas *Vaccinium myrtillus,*
- un extrait d'*Olea europaea* si le mélange de plantes de l'extrait unique ne comprend pas d'*Olea europaea,*
- de la pipérine ou un extrait de *Piper* si le mélange de plantes de l'extrait unique ne comprend pas *Piper.*

Préférentiellement, lorsque la composition comprend un extrait unique, elle comprend:

- un extrait unique obtenu à partir d'au moins *Chrysanthellum indicum, Cynara scolymus, Olea europaea* et *Vaccinium myrtillus* et *Piper,* et/ou
- de la pipérine et un extrait unique obtenu à partir d'au moins *Chrysanthellum indicum, Cynara scolymus, Olea europaea* et *Vaccinium myrtillus.*

Par extrait unique obtenu à partir de plusieurs plantes « X » ou matières première végétales « X » au sens de l'invention, on entend un ensemble de molécules obtenu à partir d'un mélange d'au moins deux plantes « X » par tout procédé adapté. On peut en particulier citer les extraits aqueux (obtenus à l'aide d'un solvant aqueux), alcooliques (obtenus à l'aide d'un solvant alcoolique) ou utilisant un solvant organique, ou à l'aide d'un corps gras naturel ou un mélange de corps gras naturels, notamment une huile végétale ou un mélange d'huiles végétales. Par solvant aqueux on entend tout solvant constitué totalement ou pour part d'eau. On peut citer ainsi l'eau elle-même, les solvants hydro-alcooliques en toute proportion ou encore les solvants constitués d'eau et d'un composé comme la glycérine ou le propylène glycol en toute proportion. Parmi les solvants alcooliques on peut citer notamment l'éthanol.

Par plante ou matière première végétale au sens de l'invention, on entend la plante entière ou la partie de plante, incluant les cultures de cellules, n'ayant pas encore subi de traitement spécifique et destinée à entrer dans la fabrication d'une préparation de plante.

L'extrait unique d'un mélange de plantes « X » peut être obtenu par tout procédé adapté, par exemple par un procédé comprenant les étapes suivantes :

- extraction solide / liquide
- séparation / pressage
- filtration
- évaporation
- séchage
- éventuellement incorporation d'additifs
- homogénéisation
- conditionnement.

On utilise préférentiellement la plante entière ou les parties aériennes de *Chrysanthellum indicum* comme matière première végétale pour obtenir l'extrait unique. L'extrait unique est réalisé préférentiellement à partir d'au moins 0,1% de plante entière ou des parties aériennes de *Chrysanthellum indicum* en poids par rapport au poids total du mélange de plantes utilisé pour réaliser l'extrait unique.

On utilise préférentiellement les feuilles ou les racines de *Cynara scolymus* comme matière première végétale pour obtenir l'extrait unique. L'extrait unique est réalisé préférentiellement à partir d'au moins 0,1% de feuilles ou de racines de *Cynara scolymus* en poids par rapport au poids total du mélange de plantes utilisé pour réaliser l'extrait unique. On utilise préférentiellement les fruits ou les feuilles de *Vaccinium myrtillus* comme matière première végétale pour obtenir l'extrait unique. L'extrait unique est réalisé préférentiellement à partir d'au moins 0,1% de fruits ou de feuilles de *Vaccinium myrtillus* en poids par rapport au poids total du mélange de plantes utilisé pour réaliser l'extrait unique. Si l'extrait unique est obtenu à partir d'un mélange de plantes comprenant *Piper,* on utilise préférentiellement les fruits ou les feuilles de *Piper nigrum,* de *Piper aduncum* et/ou de *Piper longum* comme matière première végétale pour obtenir l'extrait unique. L'extrait unique est réalisé préférentiellement à partir d'au moins 0,0001% de fruits ou de feuilles de *Piper nigrum,* et/ou de *Piper aduncum* et/ou de *Piper longum* en poids par rapport au poids total du mélange de plantes utilisé pour réaliser l'extrait unique.

L'extrait unique peut ainsi comprendre en plus des autres molécules au moins l'oleuropéine, l'hydroxytyrosol, et/ou moins de la pipérine.

De façon préférée, l'extrait unique comprend :

- au moins une molécule choisie parmi l'apigenine-7-O-glucuronide, la chrysanthelline A, la chrysanthelline B, l'acide caféique, la lutéoline, la maritimétine, l'ériodictyol, l'isookanine, l'apigénine, la lutéoline-7-O-glucoside, la maritiméine, la maréine, l'ériodictyol-7-O-glucoside, la flavomaréine, l'apigénine-8-C-α-L-arabinoside-6-C-β-D-glucoside (shaftoside), l'apigénine-6,8-C-di-β-D-glucopyranoside (vicénine-2), ou leurs analogues, préférentiellement l'apigenine-7-O-glucuronide, et
- au moins une molécule choisie parmi un acide dicaféoylquinique, un acide sulfo-monocaféoylquinique, la lutéoline, la lutéoline-7-0-glucoside, la lutéoline-7-O-glucuronide, l'apigenine-7-O-glucoside, la cynaropicrine, ou leurs analogues, préférentiellement un acide dicaféoylquinique, et
- au moins une molécule choisie parmi un acide monocaféoylquinique, la delphinidine-3-galactoside, la delphinidine-3-glucoside, la cyanidine-3-galactoside, la delphinidine-3-arabinoside, la cyanidine-3-glucoside, la petunidine-3-galactoside, la cyanidine-3-arabinoside, la petunidine-3-glucoside, la péonidine-3-galactoside, la petunidine-3-arabinoside, la péonidine-3-glucoside, la malvidine-3-galactoside, la malvidine-3-glucoside, la malvidine-3-arabinoside, ou leurs analogues, préférentiellement au moins un acide monocaféoylquinique, et
- au moins une molécule choisie parmi l'oleuropéine et l'hydroxytyrosol, et
- de la pipérine.

On utilise préférentiellement les feuilles ou les fruits d'*Olea europaea* comme matière première végétale pour obtenir l'extrait unique. L'extrait unique est réalisé préférentiellement à partir d'au moins 0,1% de feuilles ou de fruits d'*Olea europaea* en poids par rapport au poids total du mélange de plantes utilisé pour réaliser l'extrait unique.

Un mode de réalisation particulièrement adapté est une composition comprenant un extrait unique obtenu à partir de *Chrysanthellum indicum, Cynara scolymus, Vaccinium myrtillus, Piper et Olea europaea.*

La composition selon l'invention comprenant un extrait unique, lorsqu'elle est destinée à l'homme, comprend préférentiellement une quantité d'extrait unique correspondant à une administration d'au moins 0,00001g, en particulier entre 0,00001g et 0,60g d'extrait unique par kg de poids corporel de la personne à laquelle la composition est administrée et par jour. La composition selon l'invention comprenant des extraits de plantes et/ou un ou plusieurs extraits(s) unique(s), comprend donc :

- au moins une molécule choisie parmi l'apigenine-7-O-glucuronide, la chrysanthelline A, la chrysanthelline B, l'acide caféique, la lutéoline, la maritimétine, l'ériodictyol, l'isookanine, l'apigénine, la lutéoline-7-O-glucoside, la maritiméine, la maréine, l'ériodictyol-7-O-glucoside, la flavomaréine, l'apigénine-8-C-α-L-arabinoside-6-C-β-D-glucoside

(shaftoside), l'apigénine-6,8-C-di-β-D-glucopyranoside (vicénine-2), ou analogues, préférentiellement l'apigenine-7-O-glucuronide, et

- parmi un acide dicaféoylquinique, un acide sulfo-monocaféoylquinique, la lutéoline, la lutéoline-7-0-glucoside, la lutéoline-7-O-glucuronide, l'apigenine-7-O-glucoside, la cynaropicrine, ou leurs analogues, préférentiellement un acide dicaféoylquinique, et
- au moins une molécule choisie parmi un acide monocaféoylquinique, la delphinidine-3-galactoside, la delphinidine-3-glucoside, la cyanidine-3-galactoside, la delphinidine-3-arabinoside, la cyanidine-3-glucoside, la petunidine-3-galactoside, la cyanidine-3-arabinoside, la petunidine-3-glucoside, la péonidine-3-galactoside, la petunidine-3-arabinoside, la péonidine-3-glucoside, la malvidine-3-galactoside, la malvidine-3-glucoside, la malvidine-3-arabinoside, ou leurs analogues, préférentiellement au moins un acide monocaféoylquinique, et
- au moins de la pipérine, et
- et au moins une molécule choisie parmi l'oleuropéine, l'hydroxytyrosol, ou leurs analogues.

Selon un autre aspect, l'invention vise également une composition comprenant un mélange d'au moins quatre molécules, ces molécules pouvant être des molécules de synthèse et/ou des molécules naturelles notamment issues de matières premières végétales :

- au moins une molécule étant choisie parmi l'apigenine-7-O-glucuronide, la chrysanthelline A, la chrysanthelline B, l'acide caféique, la lutéoline, la maritimétine, l'ériodictyol, l'isookanine, l'apigénine, la lutéoline-7-O-glucoside, la maritiméine, la maréine, l'ériodictyol-7-O-glucoside, la flavomaréine, l'apigénine-8-C-α-L-arabinoside-6-C-β-D-glucoside (shaftoside), l'apigénine-6,8-C-di-β-D-glucopyranoside (vicénine-2), ou leurs analogues, et
- au moins une molécule étant choisie parmi un acide dicaféoylquinique, un acide sulfo-monocaféoylquinique, la lutéoline, la lutéoline-7-0-glucoside, la lutéoline-7-O-glucuronide, l'apigenine-7-O-glucoside, la cynaropicrine, ou leurs analogues, et
- au moins une molécule étant choisie parmi un acide monocaféoylquinique, la delphinidine-3-galactoside, la delphinidine-3-glucoside, la cyanidine-3-galactoside, la delphinidine-3-arabinoside, la cyanidine-3-glucoside, la petunidine-3-galactoside, la cyanidine-3-arabinoside, la petunidine-3-glucoside, la péonidine-3-galactoside, la petunidine-3-arabinoside, la péonidine-3-glucoside, la malvidine-3-galactoside, la malvidine-3-glucoside, la malvidine-3-arabinoside, ou leurs analogues, et
- au moins une molécule choisie parmi l'oleuropéine, l'hydroxytyrosol, ou leurs analogues, et
- au moins de la pipérine.

Au moins une des trois premières molécules étant une molécule de synthèse. En effet, indépendamment de la pipérine qui peut être de synthèse ou naturelle, au moins une des autres molécules est une molécule de synthèse.

Selon une variante particulièrement adaptée, le mélange comprend au moins un acide dicaféoylquinique, l'apigenine-7-O-glucuronide, un acide monocaféoylquinique, de la pipérine et l'oleuropéine. Le mélange de molécules de la composition selon l'invention peut être constitué exclusivement d'un acide dicaféoylquinique, de l'apigenine-7-O-glucuronide, d'un acide monocaféoylquinique, de pipérine et d'oleuropéine.

Selon d'autres variantes, les compositions selon l'invention peuvent comprendre un mélange d'extrait(s) et de molécule(s) d'intérêts présentés dans la présente demande (par exemple un extrait de *Chrysanthellum indicum* et au moins une molécule étant choisie parmi un acide dicaféoylquinique, un acide sulfo-monocaféoylquinique, la lutéoline, la lutéoline-7-0-glucoside, la lutéoline-7-O-glucuronide, l'apigenine-7-O-glucoside, la cynaropicrine, ou leurs analogues, et au moins une molécule étant choisie parmi un acide monocaféoylquinique, la delphinidine-3-galactoside, la delphinidine-3-glucoside, la cyanidine-3-galactoside, la delphinidine-3-arabinoside, la cyanidine-3-glucoside, la petunidine-3-galactoside, la cyanidine-3-arabinoside, la petunidine-3-glucoside, la péonidine-3-galactoside, la petunidine-3-arabinoside, la péonidine-3-glucoside, la malvidine-3-galactoside, la malvidine-3-glucoside, la malvidine-3-arabinoside, ou leurs analogues et au moins de la pipérine, etc.)

Les compositions selon l'invention sous leurs différentes variantes, peuvent être exclusivement constituées des éléments décrits (extraits de plante et/ou extrait(s) unique(s) et/ou mélange d'au moins quatre molécules), ou bien peuvent comprendre également au moins un élément supplémentaire (produits, molécules, extraits, principes actifs, excipients, etc) ajouté en plus des extraits de plante et/ou du ou des extraits uniques ou du mélange d'au moins quatre molécules, ledit élément supplémentaire pouvant être choisi parmi :

- les vitamines suivantes : B1, B2, B3, B5, B6, B8, B9, B12 C, A, D, E, K1 et K2 ;
- les composés suivants : acide obeticholique, acide corosolique, acides gras polyinsaturés de la famille des oméga 6 et/ou oméga 3, acide orotique, acide pangamique, acide para-amino-benzoïque, amygdaline, béta-glucanes, carnitine, diméthylglycine, imeglimine, isoflavones, L-arginine, oxytocine, pectine, pyridoxamine, resvératrol, viniférine, L-citrulline ;

- les oligo-éléments et minéraux suivants : arsenic, bore, calcium, cuivre, fer, fluor, iode, lithium, manganèse, magnésium, molybdène, nickel, phosphore, sélénium, vanadium, zinc ;
- les microconstituants à caractère non indispensable suivants : acide linolénique conjugué, acide lipoïque, caroténoïdes, carnitine, choline, coenzyme Q10, phytostérols, polyphénols de la famille des tannins et des lignanes, taurine ;
- des fructo-oligosaccharides, des galacto-oligosaccharides ;
- des ferments lactiques ;
- des levures, par exemple de la levure de riz rouge (*Monascus purpureus*) ;
- des champignons, par exemple le maïtaké ;
- les produits dérivés d'insectes compatibles avec le secteur alimentaire et pharmaceutique ;
- la marijuana et le haschisch ;
- des agents d'enrobage : par exemple l'hypromellose, la cellulose microcristalline, l'acide stéarique, du talc, du sucre, de la gomme laque, du povidone, de la cire d'abeille ;
- des arômes : par exemple d'arôme naturel de myrtille ou d'arôme naturel de fraise ;
- des acidifiants comme l'acide malique ;
- des antiagglomérants : par exemple le dioxyde de silicium ou le stéarate de magnésium ;
- des épaississants comme la gomme de xanthane, la silice colloïdale, les mono et diglycérides d'acides gras ;
- des stabilisants comme le phosphate de calcium ;
- des émulsifiants comme la lécithine de soja ;
- des agents de charge comme l'amidon de maïs ;
- des excipients : par exemple de la cellulose microcristalline, du stéarate de magnésium ou du phosphate dicalcique.

Les compositions selon l'invention peuvent également comprendre un ou plusieurs extraits d'au moins une des matières premières végétales suivantes et/ou une ou plusieurs molécules contenues dans au moins une des matières végétales suivantes et/ou l'extrait unique peut être obtenu également à partir d'au moins une des matières premières végétales suivantes : *Abelmoschus esculentus, Abies Alba, Abies balsamea, Abies sibirica, Acacia nilotica, Acacia senegal, Achillea millefollium, Achyranthes bidentata, Acmella oleracea, Actaea racemosa, Actinidia chinensis, Actinidia deliciosa, Adansonia digitata, Adiantum capillus-veneris, Aesculus hippocastanum, Afromomum melegueta, Agathosma betulina, Agathosma crenulata, Agathosma serratifolia, Agrimonia eupatoria, Ajuga reptans, Albizia julibrissin, Alchemilla vulgaris, Alliara petiolata, Allium ampeloprasum, Allium cepa, Allium sativum, Allium schoenoprasum, Allium ursinum, Alnus glutinosa, Aloe ferox, Aloe vera, Aloysia citriodora, Alpinia galanga, Alpinia hainanensis, Alpinia officinarum, Alpinia oxyphylla, Althaea officinalis, Ammi visnaga, Amorphophallus konjac, Ananas comosus, Andographis paniculata, Anemarrhena asphodeloides, Anethum graveolens, Angelica archangelica, Angelica dahurica, Angelica pubescens, Angelica sinensis, Antennaria diocia, Anthriscus cerefolium, Anthyllis vulneraria, Aphanizomenon flos-aquae Ralfs, Apium graveolens, Arachis hypogaea, Aralia elata, Arctium lappa, Arctium minus, Argania spinosa, Armorica rustanica, Artemisia dracunculus, Artemesia vulgaris, Ascophyllum nodosum, Aspalathus linearis, Asparagus officinalis, Astragalus membranaceus, Atractylodes lancea, Atractylodes macrocephala, Auracaria columnaris, Avena* staiva, Ayahuasca, *Baccharis genistelloides, Bacopa monnierri, Ballota nigra, Bambusa bambos, Bellis perennis, Berberis vulgaris, Beta vulgaris, Betula alleghaniensis, Betula pendula, Betula pubescens, Bixa orellana, Borago officnalis, Boswellia serrata, Brassica napus, Brassica nigra, Brassica oleracea, Brassica rapa, Bupleurum chinense, Calendula officinalis, Calluna vulgaris, Camellia sinsensis, Capsella bursa-pastoris, Capsicum annuum, Carex arenaria, Carica papaya, Carlina acaulis, Carphephorus odoratissmus, Carpinus betulus, Carthamus tinctorius, Carum carvi, Cassia fistula, Castanea sativa, Centaurea centaurium, Centaurea cyanus, Centaurium erythraea, Centella asiatica, Cerasus vulgaris, Ceratonia silliqua, Chaenomelum nobile, Chlorella vulgaris, Chondrus crispus, Chrysanthellum indicum, Cichorium intybus, Cinchona officinalis, cinchona pubescens, Cinnamomum camphora, Cinnamomum cassia, Cinnamomum verum, Cistanche salsa, Cistus incanus, Citrus aurantium, Citrus limon, Citrus maxima, Citrus medica, Citrus myrtifolia, Citrus reticulata blanco, Citrus sinsensis, Citrus paradisi, Clinopodium vulgare, Cnicus benedictus, Cochlearia officinalis, Cocos nucifera, Codonopsis pilosula, Coffea canephora, Coix lacryma-jobi var. mayyuen Stapf, Cola acuminata, Cola ballayi cornu, Cola nitida, Combretum micranthum, Commiphora mukul, Conyza canadensis, Coriandrum sativum, Cornus officinalis, Corylus avellana, Corymbia citriodora, Crataegus laevigata, Craetegus monogyna, Crithmum maritimum, Crocus sativus, Cucumis melo, Cucurbita pepo, Cuminum cyminum, Cupressus sempervirens, Cuscuta chinensis, Cyamopsis tetragonoloba, Cyathula officinalis, Cyclanthera pedata, Cydonia oblonga, Cymbopogon martini, Cymbopogon nardus, Cymbopogon winterianus, Cynara cardunculus, Cyperus rotundus, Daucus carota, Dendranthema grandiflorum, Desmodium adscendens, Dimocarpus longan, Dioscorea oppostifolia, Dioscorea villosa, Diospyros kaki Thunb., Dunaliella saliena, Echinacea augustifolia, Echinacea pallida, Echinacea purpurea, Elaegnus rhamnoides, Alettaria cardamomum, Eleutherococcus senticosus, Elymus repens, Epiobium augustifolium, Epilobium parviflorum, Equisetum arvense, Erica cinerea, Erica tetralix, Eriobotrya japonica, Eriodictyon californicum, Erodium cicutarium, Eryngium campestre, Eschscholzia californica, Eucalyptus dives Schauer, Eucalyptus globulus, Eucalyptus radiata, Eucalyptus smithii F. Muell, Eucommia*

*ulmoides, Eugenia uniflora, Eugenia jambolana, Euphrasia stricta D. Wolff Euterpe oleracea, Fagopyrum esculentum Moench, Follopia japonica, Ferula assa-foetida, Ficus carica, Filipendula ulmaria, Foeniculum vulgare Mill., Forsythia suspensa, Fragaria dodonei Ard., Frangula purshiana Cooper, Fraxinus excelsior, Fraxinus ortus, Fucus serratus, Fucus vesiculosus, Fumaria officinalis, Galeopsis segetum Neck., Galium odotarum, Galium verum, Gardenia jasminoides J. Ellis, Gastrodia elata Blume, Gelidium corneum J.V. Lamouroux, Gentiana lutea, Geranium robertianum, Geum urbanum, Ginkgo biloba, Glycine max, Glycyrrhiza glabra, Glycyrrhiza uralensis, Gracilaria gracilis, Grindelia camporum Greene, Grindelia robusta Nutt., Grindelia squarrosa Dunal, Gymnema sylvestris, Haematococcus pluvialis, Hamamemis virginiana, Harpagophytum procumbens, Harpagophytum zeyheri Decne., Hedeoma pluegioides Pers., Helianthus annuus, Helienthus tuberosus, Helichrysum arenarium, Helichrysum stoechas, Herniara glabra, Hibiscus sabdariffa, Hieracium pilosella, Himanthalia elongata, Hordeum vulgare, Houttuynia cordata Thunb., Huperzia serrata, Hyssopus officinalis, Ilex paraguariensis A. St.-Hill, Illicum verum, Impatients balsamina, Inula britannica, Inula helenium, Jasminum grandiflorum, Jasmium officinale, Juniperus communis, Justicia adhatoda, Kavalama urens, Krameria lappacea, Lagerstroemia speciosa, Laminaria digitata, Laminaria hyperborea, Lamium album, Larix decidua, Larix occidentalis, Laurus nobilis, Lavandula augustofolia, Lavandula latifolia, Ledum palustre, Leonurus cardiaca, Lepidium meyenii Walp., Lepidium sativum, Lespedeza capitata, Levisticum officinale, Lindera aggregata, Linus usitatissimum, Liquidambar styraciflua, Lotus corniculatus, Lycium chinense, Lycium barbarum, Lycopersicon esculentum, Lycopodium clavatum, Lycopus europaeus, Lythrum salicaria, Macadamia ternifolia F. muell, Macrocystis pyrifera, Magnolia officinalis, Malpighia glabra, Malus pumila, Malus domestica, Malus sylvestris, Malva sylvestris, Mangifera indica, Maranta arundinacea, Marrubium vulgare, Marsdenia cundurango, Marsdenia sylvestris, Mastocarpus stellatus, Matricaria chamomilla, Medicago sativa, Melaleuca alternifolia, Melaleuca cajuputi Powell, Melaleuca leucadendra, Melaleuca quinquenrvia, Melaleuca viridiflora, Melilotus altissimus Thuill., Melilotus officinalis, Mentha arvensis, Mentha x piperita, Menyanthes trifoliata, Mesembryanthemum crystallinum, Monarda didyma, Morinda citrifolia, Morinda officinalis, Morus alba, Morus nigra, Murraya koenigii, Musa x paradisiaca, Myrciaria dubia, Myristica flagrans Houtt., Myroxylon balsamum, Myrtus communis, Nardostachys jatamansi, Nasturtium officinale R. Br., Nelumbo nucifera Gaertn., Nepeta cataria, Nepeta tenuifolia Benth., Nigella sativa, Ocimum basilicum, Oenothera biennis, Ononis spinosa, ophiopogon japonicus, Opuntia ficus-indica, Origanum compactum Benth., Origanum majorana, Origanum vulgare, Orthosiphon aristatus, Oryza sativa, Paeonia lactiflora, Paeonia x suffruticosa Andrews, Palmaria palmata, Panax ginseng, Panax quinquefolius, Panicum miliacium, Papaver rhoeas, Parietaria officinalis, Passiflora edulis Sims, Pastinaca sativa, Paullinia cupana Kunth, Pelargonium graveolens, Perilla frutescens, Persea americana, Persicaria bistorta, Persicaria maculosa Gray, Petroselinum crispum, Peucadanum ostruthium, Peumus boldus Molina, Phaseolus vulgaris, Phellodendron amurense, Photinia melancarpa, Phyllanthus emblica, Physalis alkekengi, Phymatolithon calcareum, Picea abies, Pimenta dioca, Pimenta racemosa, Pimpinella anisum, Pimpinella major, Pimpinella saxfraga, Pinus mugo Turra, Pinus pinaster Aiton, Pinus sylvestris, Pistacia lentiscus, Plantago arenaria, Plantago lanceolata, Plantago major, Plantago ovata, Platycodon grandiflorus, Plectranthus barbatus Andrews, Pogostemom cablin, Polygala senega, Polygala sibirica, Polygala tenuifolia Willd., Polygonum aviculare, Populus nigra, Populus tremula, Populus tremuloides, Porphyra umbilicalis, Portulaca oleracea, Potentilla erecta, Primula veris, Prunella vulgaris, Prunus africana, Prunus armeniaca, Ribes nigrum, Ribes uva-crispa, Rosa canina, Rosa gallica, Rosa moschata, Rosa rubiginosa, Rosmarinus officinalis, Rubus caesius, Rubus fruticosus, Rubus idaeus, Rumex actetosa, Rumex acetosella, Rumex crispus, Rumex patienta, Ruscus aculeatus, Sachharina japonica, Saccharina latissima, Salix alba, Salix fragilis, Salix pentandra, Salix purpurea, Salvia officinalis L., Salvia officinalis subsp. lavandulifolia Gams, Salvia sclarea, Sambucus nigra, Sanguisorba officinalis, Sanicula elata Buch.-Ham. Ex D. Don, Santalum album, Santolina chamaecyparissus, Saposhnikovia divaricata, Sargassum fusiforme, Satureja hortensis, Satureja montana, Saussurea costus, Scrophularia ningpoensis Helmsl., Scutellaria baicalensis Georgi, Secale cereale, Sedum acre, Sedum roseum, Senna alexandrina Mill., Senna obustifolia, Smilax cordifolia Humb. & Bonpl., Smilax glabra Roxb., Smilax officinalis Kunth, Smilax purhampuy Ruiz, Smilax purhampuy Ruiz, Smilax regelli Killip et C.V. Morton, Smilax vanillidora Apt, Solanum melongena, Solanum tuberosum, Solidago virgaurea, Sorbus aucuparia, Spatholobus suberctus Dunn., Spinacia oleracea, Spirulina major Kützing, Spirulina maxima Geitler, Spirulina platensis Geitler, Stavhys officinalis, Stemmacantha carthamoides Dittrich, Styph! olobium japonicum, Syzgium aromaticum, Tagetes erecta, Tamarindus indica, Tanacetum parthemium, Terminalia chebula Retz., Theobroma cacao, Thymus saturejoides Coss., Thymus serpyllum, Thymus vulgaris, Thymus zygis, Tilia cordata Mill., Tilia platyphyllos Scop., Tilia tomentosa Moench, Tilia euopaea, Tribulus terrestris, Trichosanthes kirilowii Maxim., Trifolium arvense, Trifolium campestre Schreb., Trifolium pratense, Trifolium repens, Trigonella caerulea, Trigonella foenum-graecum, Tricitum aestivum, Tricitum durum Desf., Tricitum spelta L., Tricitum turgidum, Tropaeolum majus, Turnera diffusa Willd., Ulmus glabra Huds., Ulmus glabra Huds., Ulmus pumila, Ulmus rubra Muhl., Ulva lactuca, Uncaria gambir Roxb., Uncaria rhynchophylla Miq., Uncaria tomentosa DC., Undaria pinnatifida Suringar, Urtica dioca, Urtica urens, Vaccinium macrocarpon, Vaccinium oxycoccos, Vaccinium vitis-idae, Valeriana jatamansi Jones, Valeriana officinalis, Vanilla planifolia Jacks, Verbascum densiflorum Bertol., Verbascum thapsus, Verbena officinalis, Veronica officinalis, Viburnum opulus, Vigna angularis Ohwi & H. Ohashi, Vinca major, Vinca minor, Viola palustris, Viola tricolor, Vitex agnus-castus, Vitex trifolia, Vitis vinifera, Zea mays, Zingiber officinale Roscoe, Ziziphus jujuba Mill.*

Les compositions selon l'invention peuvent se présenter sous toutes formes, notamment sous forme de poudre, de gel, d'émulsion ou sous forme liquide, en particulier sous forme de comprimés, capsules, gélules, sticks, sachets, ampoules, compte-goutte ou sous forme injectable.

Les compositions selon l'invention peuvent être utilisées comme produits de nutrition ou produit de santé en particulier comme médicament.

Par produit de nutrition, on entend tous les produits ayant un effet nutritionnel et/ou physiologique, cela comprend notamment, les compléments alimentaires, les aliments, les produits diététiques, etc. Ces produits sont en particulier administrables par voie orale, gastrique ou veineuse.

Par produit de santé, on entend tous les produits ayant un effet bénéfique pour la santé, en prévention ou en traitement, que cet effet soit physiologique ou pharmacologique, notamment les médicaments, les produits pharmaceutiques. Ces produits sont en particulier administrables par voie orale, gastrique, veineuse ou cutanée.

Les compositions selon l'invention peuvent être utilisées pour prévenir et/ou lutter contre les dérèglements du métabolisme glucidique et/ou lipidique chez l'Homme ou l'animal.

Elles sont particulièrement adaptées pour prévenir et/ou lutter contre le diabète de type 2 chez l'Homme ou l'animal. En effet, elles permettent d'empêcher l'instauration d'une hyperglycémie chronique, de diminuer la glycémie à jeun et l'hémoglobine glyquée, les triglycérides circulants et hépatiques, la masse corporelle et la masse grasse, d'augmenter le HDL cholestérol (« bon cholestérol »), et d'améliorer la tolérance aux glucides ingérés et la sensibilité à l'insuline. Elles peuvent être utilisées en prévention du traitement du diabète de type 2 et en première intention, lors de l'instauration des MHD, permettant ainsi de reculer l'instauration des molécules antidiabétiques orales usuelles. Elles sont également particulièrement adaptées pour le traitement du diabète de type 2 et de ses complications, stéatose hépatique non-alcoolique (NASH) notamment, seules ou en association avec d'autres traitements pharmacologiques.

Les compositions selon l'invention peuvent également être utilisées dans la prévention et/ou la lutte contre le diabète de type 1 et/ou des maladies du foie gras non alcoolique, en particulier la stéatose hépatique non alcoolique (NASH), et/ou des pathologies cardiovasculaires, en particulier les cardiopathies coronariennes, les maladies cérébrovasculaires, les artériopathies périphériques, les thromboses veineuses profondes, et/ou des pathologies liées à une insulino-résistance comme par exemple la maladie d'Alzheimer (Bedse G et al. Front Neurosci 2015; 9:204).

Pour de telles utilisations, les compositions selon l'invention peuvent être utilisées en combinaison avec au moins un agent thérapeutique antidiabétique choisi parmi les biguanides dont la metformine, les inhibiteurs de la dipeptidyl peptidase-IV (DPP-IV), les analogues au glucagon-like peptide-1 (GLP-1), les thiazolidinediones (TZDs), les sulfonylurées, les insulines rapides et lentes, les inhibiteurs des glycosidases (acarbose, miglitol, voglibose), les inhibiteurs du sodium glucose co-transporter-2 (SGLT2), les molécules de la famille du fibranor comme l'elafibranor, ou les molécules ciblant les récepteurs nucléaires et notamment les récepteurs RORs ($\alpha$, $\beta$, $\gamma$) et Rev-Erbs ($\alpha$, $\beta$).

Les compositions selon l'invention peuvent également être utilisées pour agir sur d'autres facteurs du risque cardiovasculaire ou du syndrome métabolique.

En particulier, les compositions selon l'invention peuvent être utilisées pour prévenir et/ou lutter contre la dyslipidémie. Elles présentent notamment un effet hypocholestérolémiant et permettent de diminuer le taux de cholestérol total, le taux de LDL cholestérol, les triglycérides circulants et les triglycérides hépatiques. Elles présentent en outre une activité inhibitrice de l'HMG-CoA réductase.

Pour de telles utilisations, les compositions selon l'invention peuvent être utilisées en combinaison avec un agent thérapeutique hypolipémiant choisi parmi : les statines, les fibrates, l'acide nicotinique, les résines échangeuses d'ions, les inhibiteurs de l'absorption du cholestérol, les acides gras polyinsaturés oméga 3, le tiadénol, et les agonistes du récepteur nucléaire FXR (Farnesoid X Receptor).

Enfin, les compositions selon l'invention peuvent être utilisées spécifiquement pour prévenir ou lutter contre l'obésité et le surpoids et/ou le syndrome métabolique et/ou les problèmes pathologiques de tension artérielle.

L'invention est à présent illustrée par des exemples d'extraits et de compositions, ainsi que par des résultats d'essais démontrant l'efficacité des compositions selon l'invention, ces exemples et essais n'étant pas limitatifs.

## I. EXEMPLES

### Exemple 1 : exemple d'extrait sec de *Chrysanthellum indicum*

On soumet les parties aériennes de la plante, fraîche ou sèche, à un broyage mécanique jusqu'à obtention d'une poudre grossière. Cette poudre est ensuite soumise à une étape de macération pendant 10 à 24 heures à température ambiante dans un mélange 70/10 eau / éthanol, puis on fait subir à l'ensemble obtenu une lixiviation en continu à 50°C dans un percolateur avec un mélange 70/10 eau / éthanol, le rapport plante / extrait étant de 3/1. L'extrait obtenu est ensuite soumis à des lavages liquide / liquide à l'aide d'un solvant organique non polaire comme le di- ou le trichlorométhane. Après concentration par évaporation à basse pression à 35°C, on obtient un liquide qui, lyophilisé pendant 24 heures, donne une poudre beige soluble dans un mélange eau / alcool. Cette poudre (extrait sec) peut être

utilisée directement ou bien mélangée dans un solvant approprié avant utilisation.

Exemple 2 : exemple d'extrait sec de *Vaccinium myrtillus*

La myrtille sous forme de poudre obtenue à partir de fruits de *Vaccinium myrtillus,* est soumise à une étape de macération pendant 10 à 24 heures à température ambiante dans un mélange 30/50 eau / éthanol, puis on fait subir à l'ensemble obtenu une lixiviation en continu à 50°C dans un percolateur avec un mélange 30/50 eau / éthanol, le rapport plante / extrait étant de 10/1. L'extrait obtenu est ensuite soumis à des lavages liquide / liquide à l'aide d'un solvant organique non polaire comme le di- ou le tri-chlorométhane. Après concentration par évaporation à basse pression à 35°C, on obtient un liquide qui, lyophilisé pendant 24 heures, donne une poudre de couleur violette soluble dans un mélange eau / alcool.

Exemple 3 : exemple d'extrait sec de *Cynara scolymus*

L'artichaut sous forme de poudre obtenue à partir de feuilles de *Cynara scolymus* est soumise à une étape de macération pendant 10 à 24 heures à température ambiante dans de l'eau, puis on fait subir à l'ensemble obtenu une lixiviation en continu à 50°C dans un percolateur avec de l'eau, le rapport plante / extrait étant de 2/1. L'extrait obtenu, est ensuite soumis à des lavages liquide / liquide à l'aide d'un solvant organique non polaire comme le di- ou le tri-chlorométhane. Après concentration par évaporation à basse pression à 35°C, on obtient un liquide qui, lyophilisé pendant 24 heures, donne une poudre beige soluble dans l'eau.

Exemple 4 : exemple d'extrait sec d'*Olea europaea*

Les feuilles d'olivier entières et séchées à l'air sont broyées à -80°C à l'aide d'un broyeur à couteaux pour obtenir une poudre fine et homogène. La poudre obtenue est ensuite soumise à une étape de macération pendant 10 à 24 heures dans un mélange 70/30 eau / éthanol. L'étape est conduite en système fermé avec bullage d'azote à température ambiante, ou sous une puissance de micro-ondes de 800 watts ou sous une fréquence d'ultrasons de 20 kHz pendant 2 x 3 min. On fait ensuite subir à l'ensemble obtenu une lixiviation en continu à 50°C dans un percolateur avec un mélange 70/30 eau / éthanol, le rapport plante / extrait étant de 10/1. L'extrait obtenu est ensuite soumis à des lavages liquide / liquide à l'aide d'un solvant organique non polaire comme le di- ou le tri-chlorométhane. Après concentration par évaporation à basse pression à 35°C, on obtient un liquide qui, lyophilisé pendant 24 heures, donne une poudre de couleur verte dans un mélange eau / alcool.

Exemple 5 : exemples d'extrait unique

Le Chrysanthellum sous forme de poudre obtenue à partir des parties aériennes de Chrysanthellum *indicum,* et l'artichaut sous forme de poudre obtenue à partir des feuilles de *Cynara scolymus,* et la myrtille sous forme de poudre obtenue à partir de fruits de *Vaccinium myrtillus,* et de la poudre de fruits de *Piper nigrum,* et l'olivier sous forme de poudre obtenue à partir des feuilles de l'*Olea europaea* sont soumis à une étape de macération pendant 10 à 24 heures à température ambiante dans un mélange 40/60 eau / éthanol, puis on fait subir à l'ensemble obtenu une lixiviation en continu à 50°C dans un percolateur avec un mélange 40/60 eau / éthanol, le rapport mélange de plantes / extrait unique étant de 4 à 6 pour 1. L'extrait obtenu est ensuite soumis à des lavages liquide / liquide à l'aide d'un solvant organique non polaire comme le di- ou le tri-chlorométhane. Après concentration par évaporation à basse pression à 35°C, on obtient un liquide qui, lyophilisé pendant 24 heures, donne une poudre de couleur violette soluble dans un mélange eau / alcool.

Exemple 6 : exemple de composition selon l'invention sous forme de comprimés, comprenant quatre extraits de plantes

La composition de l'exemple 6 se présente sous forme de comprimés administrables par voie orale. Elle comprend, exprimés en pourcentage en poids, rapporté au poids total de la composition, 30,1% d'extrait sec de parties aériennes de *Chrysanthellum indicum,* 30,1% d'extrait sec de feuilles de *Cynara scolymus,* 3,0% d'extrait sec de fruits de *Vaccinium myrtillus* et 0,3% d'extrait sec de fruits de *Piper nigrum.* Elle comprend également des excipients, en particulier de la cellulose microcristalline et du stéarate de magnésium.

La composition pour 3 comprimés est indiquée dans le **Tableau 1** suivant.

***Tableau 1. Exemple de composition sous forme de comprimés***

| Liste des ingrédients | Pour 3 comprimés |
|---|---|
| Extrait sec de parties aériennes de *Chrysanthellum indicum* | 600 mg |
| Extrait sec de feuilles de *Cynara scolymus* | 600 mg |
| Extrait sec de fruits de *Vaccinium myrtillus* | 60 mg |
| Extrait sec de fruits de *Piper nigrum* | 6 mg |
| Cellulose microcristalline | 700 mg |
| Stéarate de magnésium | 26 mg |

Exemple 7 : exemple de composition selon l'invention sous forme de gélules, comprenant cinq extraits de plantes

La composition de l'exemple 7 se présente sous forme de gélules administrables par voie orale. Elle comprend, exprimés en pourcentage en poids, rapporté au poids total de la composition, 37,0% d'extrait sec de parties aériennes de *Chrysanthellum indicum,* 37,0% d'extrait sec de feuilles de *Cynara scolymus,* 3,7% d'extrait sec de fruits de *Vaccinium myrtillus,* 0,004% d'extrait sec de fruits de *Piper nigrum,* et 22,2% d'extrait sec de feuilles d'*Olea europaea.*

La composition pour 3 gélules est indiquée dans le **Tableau 2** suivant.

***Tableau 2. Exemple de composition sous forme de gélules***

| Liste des ingrédients | Pour 3 gélules |
|---|---|
| Extrait sec de parties aériennes de *Chrysanthellum indicum* | 200 mg |
| Extrait sec de feuilles de *Cynara scolymus* | 200 mg |
| Extrait sec de fruits de *Vaccinium myrtillus* | 20 mg |
| Extrait sec de fruits de *Piper nigrum* | 0,02 mg |
| Extrait sec de feuilles d'*Olea europaea* | 120 mg |

Exemple 8 : exemple de composition selon l'invention sous forme de comprimés, comprenant cinq extraits de plantes

La composition de l'exemple 8 se présente sous forme de comprimés administrables par voie orale. Elle comprend, exprimés en pourcentage en poids, rapporté au poids total de la composition, 22,0% d'extrait sec de parties aériennes de *Chrysanthellum indicum,* 22,0% d'extrait sec de feuilles de *Cynara scolymus,* 2,2% d'extrait sec de fruits de *Vaccinium myrtillus,* 13,2% d'extrait sec de feuilles d'*Olea europaea,* et 0,2% d'extrait sec de fruits de *Piper nigrum.* La composition comprend également en plus du mélange du zinc, les vitamines B9, PP, B5, H, B12, D, B6, B2, B2 et du chrome. Elle comprend également des excipients en particulier du phosphate dicalcique, de la cellulose microcristalline et du stéarate de magnésium.

La composition pour 1 comprimé est indiquée dans le **Tableau 3** suivant.

***Tableau 3. Exemple de composition sous forme de comprimés***

| Liste des ingrédients | En mg pour 1 comprimé | Valeur Nutritionnelle de Référence pour 1 comprimé |
|---|---|---|
| Extrait sec de parties aériennes de *Chrysanthellum indicum* | 220,0 | - |
| Extrait sec de feuilles de *Cynara scolymus* | 220,0 | - - |
| Extrait sec de fruits de *Vaccinium myrtillus* | 22,0 | - |
| Extrait sec de feuilles d'*Olea europaea* | 132,0 | - |
| Extrait sec de fruits de *Piper nigrum* | 2,0 | - |
| Phosphate dicalcique | 198,0 | - |
| Cellulose microcristalline | 153,44 | - |

(suite)

| Liste des ingrédients | En mg pour 1 comprimé | Valeur Nutritionnelle de Référence pour 1 comprimé |
|---|---|---|
| Stéarate de magnésium | 20,0 | |
| Bisglycinate de zinc | 12,99 | 33% |
| Vitamine B9 - acide folique | 7,30 | 33% |
| Vitamine PP - nicotinamide | 5,30 | 33,1% |
| Vitamine B5 - calcium pantothénate | 2,24 | 33,3 |
| Vitamine H - biotine | 1,66 | 33,2% |
| Vitamine B12 | 0,83 | 33,2% |
| Vitamine D3 | 0,64 | 32% |
| Vitamine B6 - pyridoxine chlorhydrate | 0,56 | 32,9% |
| Vitamine B1 - thiamine chlorhydrate | 0,48 | 32,7% |
| Vitamine B2 - riboflavine | 0,45 | 32,1% |
| Picolinate de chrome | 0,11 | 32,5% |

Exemple 9 : exemple de composition selon l'invention sous forme de comprimés, comprenant quatre extraits de plantes

La composition de l'exemple 9 se présente sous forme de comprimés administrables par voie orale. Elle comprend, exprimés en pourcentage en poids, rapporté au poids total de la composition, 23,9% d'extrait sec de plantes entières de *Chrysanthellum indicum,* 23,9% d'extrait sec de feuilles de *Cynara scolymus,* 23,9% d'extrait sec de fruits de *Vaccinium myrtillus* et 0,2% d'extrait sec de fruits de *Piper nigrum.* La composition comprend également de la vitamine B1, de la vitamine B3, de l'acide pantothénique (vitamine B5), du zinc et du chrome. A titre d'excipients, elle comprend de la cellulose microcristalline et du phosphate dicalcique. A titre d'agents d'enrobage, elle comprend du talc, du sucre, de la gomme laque, du povidone et de la cire d'abeille.

La composition pour 3 comprimés est indiquée dans le **Tableau 4** suivant.

***Tableau 4. Exemple de composition sous forme de comprimé***

| Liste des ingrédients | Pour 3 comprimés | Valeur Nutritionnelle de Référence pour 3 comprimés |
|---|---|---|
| Extrait sec de fruits de *Vaccinium myrtillus* | 600 mg | - |
| Extrait sec de plantes entières de *Chrysanthellum indicum* | 600 mg | - |
| Extrait sec de feuilles de *Cynara scolymus* | 600 mg | - |
| Extrait sec de fruits de *Piper nigrum* | 5 mg | - |
| Vitamine B1 | 1,1 mg | 100% |
| Vitamine B3 (hexanicotinate d'inositol) | 16 mg (acide nicotinique) | 100% |
| Acide pantothénique (vitamine B5) | 6 mg | 100% |
| Zinc (bisglycinate de zinc) | 5 mg | 50% |
| Chrome (picolinate de chrome) | 40 μg | 100% |
| Cellulose microcristalline | 600 mg | - |
| Phosphate dicalcique | 75 mg | - |

Exemple 10 : exemple de composition selon l'invention sous forme de poudre à reconstituer dans l'eau, conditionnée sous forme de sticks, comprenant quatre extraits de plantes

La composition de l'exemple 10 se présente sous forme de poudre à reconstituer dans de l'eau, conditionnée sous forme de sticks administrables par voie orale. Elle comprend, exprimés en pourcentage en poids, rapporté au poids total de la composition, 36,3% d'extrait sec de plantes entières de Chrysanthellum *indicum,* 24,2% d'extrait sec de racines de *Cynara scolymus,* 36,3% d'extrait sec de fruits de *Vaccinium myrtillus* et 2,4% d'extrait sec de fruits de *Piper longum.* La composition comprend également de la vitamine B1, de la vitamine B3, de l'acide pantothénique (vitamine B5), du zinc et du chrome. A titre d'arôme, elle comprend un arôme naturel de myrtille. A titre d'acidifiant, elle comprend de l'acide malique. A titre d'antiagglomérant, elle comprend du dioxyde de silicium, à titre d'épaississant, de la gomme xanthane, et à titre de stabilisant, du phosphate de calcium.

Sa composition est indiquée dans le **Tableau 5** suivant.

***Tableau 5. Exemple de composition sous forme de poudre à reconstituer dans de l'eau et conditionnée sous forme de sticks***

| Liste des ingrédients | Pour 3 sticks | Valeur Nutritionnelle de Référence pour 3 sticks |
|---|---|---|
| Extrait sec de fruits de *Vaccinium myrtillus* | 1500 mg | - |
| Extrait sec de plantes entières de *Chrysanthellum indicum* | 1500 mg | - |
| Extrait sec de racines de *Cynara scolymus* | 1000 mg | - |
| Extrait sec de fruits de *Piper longum* | 100 mg | - |
| Vitamine B1 | 1,1 mg | 100% |
| Vitamine B3 (hexanicotinate d'inositol) | 16 mg (acide nicotinique) | 100% |
| Acide pantothénique (vitamine B5) | 6 mg | 100% |
| Zinc (bisglycinate de zinc) | 5 mg | 50% |
| Chrome (picolinate de chrome) | 40 $\mu$g | 100% |

Exemple 11 : exemple de composition selon l'invention sous forme de poudre à reconstituer dans l'eau, conditionnée sous forme de sticks

La composition de l'exemple 11 se présente sous forme de poudre à reconstituer dans de l'eau, conditionnée sous forme de sticks administrables par voie orale. Elle comprend, exprimés en pourcentage en poids, rapporté au poids total de la composition, 37,0% d'extrait sec de parties aériennes de *Chrysanthellum indicum,* 37,0% d'extrait sec de feuilles de *Cynara scolymus,* 3,7% d'extrait sec de fruits de *Vaccinium myrtillus,* 0,2% d'extrait sec de fruits de *Piper nigrum,* et 22,2% d'extrait sec de feuilles d'*Olea europaea.* La composition comprend également de la vitamine B12 et du chrome. A titre d'arôme, elle comprend un arôme naturel de fraise. A titre d'antiagglomérant, elle comprend du dioxyde de silicium. Cette composition ne comprend pas d'antiagglomérant et d'épaississant.

La composition d'un tel produit est indiquée dans le **Tableau 6** suivant.

***Tableau 6. Exemple de composition sous forme de poudre à reconstituer dans de l'eau et conditionnée sous forme de sticks***

| Liste des ingrédients | Pour 3 sticks | Valeur Nutritionnelle de Référence pour 3 sticks |
|---|---|---|
| Extrait sec de fruits de *Vaccinium myrtillus* | 120 mg | - |
| Extrait sec de parties aériennes de *Chrysanthellum indicum* | 1200 mg | - |
| Extrait sec de feuilles de *Cynara scolymus* | 1200 mg | - |
| Extrait sec de fruits de *Piper nigrum* | 6 mg | - |
| Extrait sec de feuilles d'*Olea europaea* | 720 mg | - |

(suite)

| Liste des ingrédients | Pour 3 sticks | Valeur Nutritionnelle de Référence pour 3 sticks |
|---|---|---|
| Vitamine B3 (hexanicotinate d'inositol) | 2,5 μg | 100% |
| Chrome (picolinate de chrome) | 20 μg | 50% |

Exemple 12 : exemple de composition selon l'invention sous forme de gélules

La composition de l'exemple 12 se présente sous forme de gélules administrables par voie orale. Elle comprend, exprimés en pourcentage en poids, rapporté au poids total de la composition, 31,6% d'extrait sec de parties aériennes de *Chrysanthellum indicum,* 47,4% d'extrait sec de racines de *Cynara scolymus,* 15,8% d'extrait sec de fruits de *Vaccinium myrtillus* et 2,6% de pipérine. La composition comprend également de la vitamine B3 et du zinc. A titre d'émulsifiant, elle comprend de la lécithine de soja issue de la filière non OGM, à titre d'épaississants, de la silice colloïdale et des mono- et diglycérides d'acides gras. La capsule est une gélatine de poisson, avec de la glycérine et un colorant, l'oxyde de fer rouge.

La composition d'un tel produit est indiquée dans le **Tableau 7** suivant.

***Tableau 7. Exemple de composition sous forme de gélules***

| Liste des ingrédients | Pour 2 gélules | Valeur Nutritionnelle de Référence pour 2 gélules |
|---|---|---|
| Extrait sec de fruits de *Vaccinium myrtillus* | 100 mg | - |
| Extrait sec de parties aériennes de *Chrysanthellum indicum* | 200 mg | - |
| Extrait sec de racines de *Cynara scolymus* | 300 mg | - |
| Pipérine | 15 mg | - |
| Vitamine B3 | 8 mg (acide nicotinique) | 50% |
| Zinc (gluconate de zinc) | 10mg | 100% |

Exemple 13 : exemple de composition selon l'invention sous forme de gélules

La composition de l'exemple 13 se présente sous forme de gélules administrables par voie orale. Elle comprend, exprimés en pourcentage en poids, rapporté au poids total de la composition, 10,4% d'extrait sec de plantes entières de *Chrysanthellum Indicum,* 20,7% d'extrait sec de feuilles de *Cynara scolymus,* 62,1% d'extrait sec de feuilles de *Vaccinium myrtillus* et 2,6% de pipérine. A titre d'agent de charge, elle comprend de l'amidon de maïs. A titre d'antiagglomérants, elle comprend du dioxyde de silicium et du stéarate de magnésium. La gélule est d'origine végétale.

La composition d'un tel produit est indiquée dans le **Tableau 8** suivant.

***Tableau 8. Exemple de composition sous forme de gélules***

| Liste des ingrédients | Pour 1 gélule | Valeur Nutritionnelle de Référence pour 1 gélule |
|---|---|---|
| Extrait sec de feuilles de *Vaccinium myrtillus* | 300 mg | - |
| Extrait sec de plantes entières de *Chrysanthellum indicum* | 50 mg | - |
| Extrait sec de feuilles de *Cynara scolymus* | 100 mg | - |
| Pipérine | 15 mg | - |

Exemple 14 : exemple de composition selon l'invention sous forme de gélules comprenant un extrait unique

La composition de l'exemple 14 se présente sous forme de gélules administrables par voie orale. Elle comprend un extrait unique hydro-alcoolique d'un mélange de poudre obtenu à partir des parties aériennes de *Chrysanthellum indicum,* de feuilles de *Cynara scolymus,* de fruits de *Vaccinium myrtillus,* de fruits de *Piper nigrum* et de feuilles d'*Olea europaea.* Le ratio entre les trois plantes est de 1 / 1 / 0,1 / 0,0001 / 0,6.

L'extrait unique du mélange de plantes est obtenu par un procédé comprenant les étapes suivantes :

- extraction solide / liquide
- séparation / pressage
- filtration
- évaporation
- séchage
- éventuellement incorporation d'additifs
- homogénéisation
- conditionnement.

Exemple 15 : exemples de composition selon l'invention sous forme de comprimés comprenant des molécules de synthèse ou obtenues à partir de matières premières végétales

La composition de l'exemple 15 se présente sous forme de comprimés pelliculés administrables par voie orale. Elle comprend comme substances actives pour 1 comprimé : 50 mg d'apigenine-7-O-glucuronide, 50 mg d'acide acide dicaféoylquinique, 100 mg d'acide monocaféoylquinique, 10 mg de pipérine et 250 mg d'oleuropéine. Les autres composants utilisés comme excipients sont : amidon prégélatinisé, carboxyméthylamidon sodique (type A), acide stéarique, povidone K90, silice colloïdale anhydre.

Les substances actives peuvent être de synthèse ou issues de matières premières végétales ou issues d'extraits végétaux par purification par chromatographie en phase liquide à haute performance.

II. EVALUATION IN VIVO DE l'EFFICACITE DE LA COMPOSITION

Des expérimentations *in vivo* sur des souris ont été réalisées pour démontrer les effets des compositions selon l'invention, en particulier sur la glycémie à jeun, l'hémoglobine glyquée, la tolérance aux glucides, l'insulino-sensibilité, la masse corporelle et la masse grasse, et sur les lipides circulants et hépatiques. De même, des évaluations en biologie moléculaire ont été réalisées. Enfin, les compositions ont été comparées à des traitements pharmacologiques de référence déjà sur le marché ou en cours de développement.

Les expérimentations ont été réalisées sur des souris db/db. Les souris db/db présentent une mutation des récepteurs à la leptine induisant un dérèglement de la signalisation cellulaire de cette dernière. Les récepteurs à la leptine sont hautement exprimés au niveau de l'hypothalamus. Les souris présentant une mutation de ces récepteurs ne peuvent pas réguler efficacement leurs stocks énergétiques. Il en résulte une insulinémie élevée dès les premiers jours de la vie (10 - 14 jours), et une obésité à partir de 3 à 4 semaines avec une augmentation de la glycémie. Ces souris sont insulino-résistantes, hypertriglycéridémiques, et intolérantes au glucose. Elles constituent un modèle pertinent et prédictif notamment pour l'étude de l'insulino-sensibilité, de la triglycéridémie, du diabète de type 2 et d'une de ses complications, la stéatose hépatique non alcoolique (NASH) (Aileen JF King Br J Pharmacol 2012; 166(3):877-894 ; Sanches SC et al. Biomed Res Int 2015).

11.1 Essais A

Le temps expérimental était de 9 semaines avec un « Run-in » d'1 semaine suivi de 8 semaines de complémentation avec les extraits végétaux et une composition X. Les souris males étaient âgés de 10 semaines en début de traitement.

9 compositions X ont été testées. Ces compositions étaient directement intégrées dans l'alimentation des

rongeurs, cela permet de s'assurer de son efficacité « multicibles » et de son utilisation à grande échelle, les injections intraveineuses ou intrapéritonéales étant limitées à un faible nombre de personnes étant donné leur mode d'administration. Cela évite également les gavages quotidiens qui altèrent différents processus physiologiques.

Les compositions testées étaient les suivantes :

- C1 : *Chrysanthellum indicum* (plante entière) + pipérine (respectivement 1% et 0,1% de l'alimentation) ;
- C2 : *Cynara scolymus* (feuilles) + pipérine (respectivement 1% et 0,1% de l'alimentation) ;
- C3: *Vaccinium myrtillus* (fruits) + pipérine (respectivement 1% et 0,1% de l'alimentation) ;
- C4 : *Chrysanthellum indicum* (plante entière) + *Cynara scolymus* (feuilles) + *Vaccinium myrtillus* (fruits) + pipérine (respectivement 1%, 1%, 1% et 0,1% de l'alimentation) ;
- C5 : pipérine (0,1% de l'alimentation) ;
- C6 : *Chrysanthellum indicum* (plante entière) + *Cynara scolymus* (feuilles) + *Vaccinium myrtillus* (fruits) (respectivement 1%, 1% et 1% de l'alimentation) ;
- C7 : *Chrysanthellum indicum* (plante entière) + *Cynara scolymus* (feuilles) + *Vaccinium myrtillus* (fruits) + pipérine (respectivement 1%, 1%, 1%, 0,001% de l'alimentation) ;
- C8 : *Olea europaea* (feuilles) (0,6% de l'alimentation) ;
- C9 : *Chrysanthellum indicum* (plante entière) + *Cynara scolymus* (feuilles) + *Vaccinium myrtillus* (fruits) + pipérine + *Olea europaea* (feuilles) (respectivement 1%, 1%, 1%, 0,001%, 0,6% de l'alimentation).

Par pipérine, on entend soit de la pipérine synthétique, soit un extrait de *Piper* standardisé à 95% en pipérine. Pour les autres végétaux, il s'agissait d'extraits secs obtenus à partir de matières premières végétales.

Les expérimentations se sont déroulées en plusieurs étapes. Par conséquent, plusieurs groupes « Contrôle *ad libitum »* ont été réalisés. Les résultats issus de ces groupes ont été poolés. Les compositions C1, C2, C3, C4, C5, C8 et C9 ont induit une diminution similaire de la prise alimentaire. Par conséquent un groupe « Contrôle *Per Fed »,* c'est-à-dire consommant la même quantité journalière de nourriture que les groupes C1, C2, C3, C4, C5, C8 et C9, a été réalisé de manière à pouvoir comparer les résultats à prise alimentaire équivalente.

Les évaluations expérimentales ont notamment porté sur :

- La mesure de la masse corporelle ;
- La mesure de la glycémie à jeun ;
- L'évolution de la glycémie lors d'un test oral de tolérance aux glucides. Après gavage en amidon (3 g/kg) à jeun, l'évolution de la glycémie en réponse à l'amidon était mesurée à la queue par biopsie juste avant le gavage (t0), puis après 30, 60, 90 et 120 minutes. L'aire sous la courbe (AUC = aera under the curve) était calculée. Une augmentation de l'AUC traduit une intolérance aux glucides, une diminution traduit une amélioration de la tolérance aux glucides.

Pour les compositions C7 à C9, un test de sensibilité à l'insuline a également été réalisé. Ce test consistait en une injection intrapéritonéale d'insuline (2 U/kg) à jeun. L'évolution de la glycémie en réponse à l'injection d'insuline était mesurée à la queue par biopsie juste avant l'injection (t0), puis après 30, 60, 90 et 120 minutes. L'aire sous la courbe (AUC = aera under the curve) était calculée. Une diminution de l'AUC traduit une meilleure réponse à l'injection d'insuline et donc une amélioration de la sensibilité à l'insuline. A l'inverse, une augmentation de l'AUC traduit une moins bonne sensibilité à l'insuline et donc une insulino-résistance.

Les évaluations présentées ont été réalisées juste avant la complémentation (t = 0) et en fin de complémentation (t = 8 semaines).

Les résultats obtenus sont présentés dans des tableaux :

- **Tableau 9** pour l'effet sur la sensibilité à l'insuline après 8 semaines de traitement,
- **Tableau 10** pour l'effet sur la glycémie à jeun après 8 semaines de traitement,
- **Tableau 11** pour l'effet sur la tolérance aux glucides après 8 semaines de traitement,
- **Tableau 12** pour l'effet sur la masse corporelle après 8 semaines de traitement.

Sensibilité à l'insuline

**Tableau 9. Effet des compositions sur la sensibilité à l'insuline après 8 semaines de traitement**

| Compositions | AUC (semaine 8 - semaine 0, en mg×min/dL) (moyenne ± SEM) |
|---|---|
| ***Contrôle ad libitum*** | 19 186 ± 3460 |
| ***C7*** | 2082 ± 2082 ** |
| ***C8*** | 8970 ± 2959 |
| ***C9*** | -6520 ± 4685** |
| Valeurs moyennes ± SEM. Contrôle *ad libitum,* n = 20 ; C7, n = 11 ; C8, n = 10 ; C9, n = 9. Composition *versus* Contrôle, Test *t* de Student pour données non appariées, ** $p < 0.01$. | |

Les résultats présentés dans le **Tableau 9** sont également illustrés sur la **Figure 1**. Ces résultats montrent un effet très significatif et important des compositions C7 et C9 sur la sensibilité à l'insuline. L'amélioration de la sensibilité à l'insuline avec les compositions C7 et C9 selon l'invention est particulièrement intéressante en prévention et en traitement du diabète de type 2 et de ses complications. En effet, l'insulino-résistance est un des mécanismes majeurs de progression du diabète de type 2 (Samuel VT et al. Cell 2012; 148:852-71). Par ailleurs, en agissant sur ce paramètre, les compositions C7 et C9 selon l'invention sont particulièrement adaptées dans les autres pathologies dont une des causes importantes est l'insulino-résistance. C'est en particulier le cas d'une des complications du diabète de type 2, la stéatose hépatique non-alcoolique (NASH ; Samuel VT et al. Cell 2012; 148:852-71), et de la maladie d'Alzheimer (Bedse G et al. Front Neurosci 2015; 9:204).

Glycémie à jeun

**Tableau 10**. **Effet des compositions sur la glycémie à jeun après 8 semaines de traitement**

| Compositions | Glycémie à jeun (en mg/dL) |
|---|---|
| ***Contrôle ad libitum*** | 534 ± 10 |
| ***Contrôle Per Fed*** | 518,2 ± 38 |
| ***C1*** | 410,4 ± 33* |
| ***C2*** | 448 ± 34 |
| ***C3*** | 384 ± 40* |
| ***C4*** | 335 ± 38** |
| ***C5*** | 449 ± 11 |
| ***C6*** | 536 ± 15 |
| ***C7*** | 571 ± 14* |
| ***C8*** | 400 ± 61 |
| ***C9*** | 220 ± 78** |
| Valeurs moyennes ± SEM. Contrôle *ad libitum,* n = 32 ; Contrôle *Per Fed,* n = 10 ; C1, n = 9 ; C2, n = 9 ; C3, n = 9 ; C4, n = 8 ; C5, n = 9 ; C6, n = 14 ; C7, n = 12 ; C8, n = 12 ; C9, n = 9. Composition *versus* Contrôle, Test *t* de Student pour données non appariées, $^*p < 0.05$, $^{**}p < 0.01$. | |

Les compositions C1, C2, C3, C4, C5, C8 et C9 ont provoqué une diminution de la prise alimentaire. Par conséquent un groupe *Per Fed* consommant la même quantité de nourriture a été réalisé. Les résultats montrent que la restriction calorique imposée aux animaux n'a eu aucun effet sur la glycémie à jeun (comparaison Contrôle ad libitum *versus* Contrôle Per Fed). Les compositions C1, C3, C4 et C9 ont induit une diminution significative de la glycémie à jeun, l'effet le plus marqué étant observé avec les compositions C4 (*Chrysanthellum indicum 1%, Cynara scolymus 1%, Vaccinium myrtillus 1%,* pipérine 0,1%) et C9 (*Chrysanthellum indicum* 1%, *Cynara scolymus* 1%, *Vaccinium myrtillus*

1%, pipérine 0,001%, *Olea europaea* 0,6%).

La composition C6 (*Chrysanthellum indicum* 1%, *Cynara scolymus* 1%, *Vaccinium myrtillus* 1%) n'a eu aucun effet sur la glycémie à jeun. L'ajout de la pipérine (0,1%), alors même que celle-ci (C5) n'induisait pas de diminution significative de la glycémie à jeun, à la combinaison C6 pour obtenir la combinaison C4, a permis de façon surprenante de baisser significativement et fortement la glycémie à jeun. De même, l'ajout d'*Olea europaea* (0,6% ; C8) à la combinaison C7 (*Chrysanthellum indicum* 1%, *Cynara scolymus* 1%, *Vaccinium myrtillus* 1%, pipérine 0,001%) pour obtenir la composition C9 a permis également de baisser fortement la glycémie à un niveau quasiment similaire à celui de souris non diabétiques.

Tolérance aux glucides

**Tableau 11. Effet des compositions sur la tolérance aux glucides après 8 semaines de traitement**

| Compositions | AUC (semaine 8 - semaine 0, en mgxmin/dL) (moyenne ± SEM) |
|---|---|
| ***Contrôle ad libitum*** | 10 954 ± 2000 |
| ***Contrôle Per Fed*** | 20 355 ± 6554 |
| ***C1*** | 3367 ± 3039* |
| ***C2*** | 1901 ± 3949* |
| ***C3*** | -2418 ± 3260** |
| ***C4*** | -11 537 ± 4168** |
| ***C5*** | 3610 ± 2086* |
| ***C6*** | 13 068 ± 3064 |
| ***C7*** | 8639 ± 2844 |
| ***C8*** | -8889 ± 5436** |
| ***C9*** | -24 568 ± 10 440** |
| Valeurs moyennes ± SEM. Contrôle *ad libitum,* n = 22 ; Contrôle *Per Fed,* n = 10 ; C1, n = 9 ; C2, n = 8 ; C3, n = 10 ; C4, n = 8 ; C5, n = 9 ; C6, n = 14 ; C7, n = 12 ; C8, n = 10 ; C9, n = 9. AUC : « aera under the curve » (aire sous la courbe). Composition *versus* Contrôle, Test *t* de Student pour données non appariées, * $p < 0.05$, ** $p < 0.01$. | |

Les compositions C1, C2, C3, C4, C5, C8 et C9 ont provoqué une diminution de la prise alimentaire. Par conséquent un groupe *Per Fed* consommant la même quantité de nourriture a été réalisé. Les résultats montrent que la restriction calorique imposée aux animaux n'a eu aucun effet sur la tolérance aux glucides (comparaison Contrôle ad libitum *versus* Contrôle Per Fed). Les compositions C1, C2, C3, C4, C5, C8 et C9 ont induit une diminution significative de l'AUC, ce qui traduit une amélioration de la tolérance aux glucides. L'effet le plus marqué est obtenu avec les combinaisons C4 et C9.

Masse corporelle

**Tableau 12. Effet des compositions sur la masse corporelle après 8 semaines**

| Compositions | Masse corporelle (en g, moyenne ± SEM) |
|---|---|
| ***Contrôle ad libitum*** | 36,9 ± 1,2 |
| ***Contrôle Per Fed*** | 30,6 ± 1,2 |
| ***C1*** | 33,9 ± 2,4 |
| ***C2*** | 37,4 ± 2,2* |
| ***C3*** | 37,0 ± 2,7* |

(suite)

| Compositions | Masse corporelle (en g, moyenne ± SEM) |
|---|---|
| ***C4*** | 36,3 ± 1,7* |
| ***C5*** | 32,7 ± 1,5 |
| ***C6*** | 29,3 ± 1,8*** |
| ***C7*** | 34,5 ± 1,6 |
| ***C8*** | 26,9 ± 2,0 |
| ***C9*** | 25,0 ± 2,1* |
| Valeurs moyennes ± SEM. Contrôle *ad libitum,* n = 34 ; Contrôle *Per Fed,* n = 10 ; C1, n = 9 ; C2, n = 9 ; C3, n = 9 ; C4, n = 8 ; C5, n = 9 ; C6, n = 14 ; C7, n = 12 ; C8, n = 10 ; C9, n = 9. Composition *versus* Contrôle, Test *t* de Student pour données non appariées, *p < 0.05, **p < 0.01, ***p < 0,0001. | |

Les compositions C1, C2, C3, C4, C5, C8 et C9 ont provoqué une diminution de la prise alimentaire. Par conséquent un groupe *Per Fed* consommant la même quantité de nourriture a été réalisé. Les résultats montrent que la restriction calorique imposée aux animaux a induit une diminution significative de la masse corporelle (comparaison Contrôle ad libitum *versus* Contrôle Per Fed, p < 0,05). Les compositions C2, C3, C4, C6 et C9 ont également induit une diminution significative de la masse corporelle. Alors que la combinaison C6 n'avait aucun effet sur la glycémie à jeun, celle-ci a induit une baisse importante et significative de la masse corporelle lui conférant des propriétés intéressantes dans la prévention et le traitement de l'obésité. La combinaison C9 a eu l'effet le plus marqué.

Effet synergique

Par ailleurs, l'effet synergique a été évalué selon la méthode de Colby SR décrite dans « Calculation of the synergistic and antagonitic responses of herbicide combinations » Weeds, 1967, 15:20-22. Cette méthode a notamment été utilisée dans le brevet EP03812880. Pour chaque combinaison, le facteur de synergie a été calculé. Un facteur > 1 indique l'existence d'un effet synergique. Un facteur < 1 indique l'existence d'un antagoniste. Les calculs effectués sont :

Taux d'efficacité attendu = A + B – (A * B / 100)

Facteur de synergie (FS) = (1 * taux d'efficacité observé (%)) / taux d'efficacité attendu (%)

Les calculs ont porté sur les combinaisons suivantes :

- *Calcul de FS pour la combinaison Chrysanthellum indicum 1% / Cynara scolymus 1% / Vaccinium myrtillus 1% / pipérine 0,1% (composition C4) où A = Chrysanthellum indicum 1% / Cynara scolymus 1% / Vaccinium myrtillus 1% (C6) et B = pipérine (C5)* ;
- *Calcul de FS pour la combinaison Chrysanthellum indicum 1% / Cynara scolymus 1% / Vaccinium myrtillus 1% / pipérine 0,001% / Olea europaea 0,6% (composition C9) où A = Chrysanthellum indicum 1% / Cynara scolymus 1% / Vaccinium myrtillus 1% / pipérine 0,001% (composition C7) et B = Olea europaea 0,6% (composition C8).*

Le **Tableau 13** ci-après donne les résultats pour la combinaison C6 + C5 = C4.

**Tableau 13. Facteurs de synergie pour la combinaison C6 + C5 = C4**

| | Taux d'efficacité observé (en % du groupe contrôle) | | | Taux d'efficacité attendu avec C4 | Facteur de synergie |
|---|---|---|---|---|---|
| | C6 | C5 | C4 | | |
| Glycémie à jeun après 8 semaines de traitement | +2,6% | -13,3% | -35,3% | -11,0% | 3,20 |

(suite)

| | Taux d'efficacité observé (en % du groupe contrôle) | | | Taux d'efficacité attendu avec C4 | Facteur de synergie |
|---|---|---|---|---|---|
| | C6 | C5 | C4 | | |
| Test oral de tolérance aux glucides, AUC (semaine 8 - semaine 0) | +9,5% | -82,3% | -156,7% | -82,3% | 1,94 |

Les résultats présentés dans le **Tableau 13** sont illustrés sur les **Figures 2** et **3**. Un effet synergique important avec la composition C4 selon l'invention est démontré, à la fois sur la glycémie à jeun et sur la tolérance aux glucides (AUC).

Le **Tableau 14** ci-dessous donne les résultats pour la combinaison C7 + C8 = C9.

**Tableau 14. Facteurs de synergie pour la combinaison C7 + C8 = C9**

| | Taux d'efficacité observé (en % du groupe contrôle) | | | Taux d'efficacité attendu avec C9 | Facteur de synergie |
|---|---|---|---|---|---|
| | C7 | C8 | C9 | | |
| Glycémie à jeun après 8 semaines de traitement | +5,7% | -26,1% | -59,4% | -21,9% | 2,71 |
| Test oral de tolérance aux glucides, AUC (semaine 8 - semaine 0) | +4,6% | -204,5% | -388,7% | -209,3% | 1,86 |
| Test oral de sensibilité à l'insuline, AUC (semaine 8 - semaine 0) | -89,1% | -53,2% | -129,3% | -94,9% | 1,36 |

Les résultats présentés dans le **Tableau 14** sont illustrés dans les **Figures 4, 5** et **6.** Un effet synergique important avec la composition C9 selon l'invention est démontré, à la fois sur la glycémie à jeun, sur la tolérance aux glucides (AUC) et la sensibilité à l'insuline (AUC). Et cela alors même que la composition C7 avait déjà un effet significatif et important sur l'amélioration de la sensibilité à l'insuline.

11.2 Essais B

Une autre composition, C10, a été mise au point et testée *in vivo* : *Chrysanthellum indicum* (partie aérienne, extrait sec) + *Cynara scolymus* (feuilles, extrait sec) + *Vaccinium myrtillus* (fruits, extrait sec) + *Piper nigrum* (fruits, extrait sec) + *Olea europaea* (feuilles, extrait sec).

Plus précisément, la composition C10 a été testée *in vivo* en comparaison à la metformine, la molécule thérapeutique de référence dans le traitement du diabète de type 2, sur le même modèle que les Essais A (souris db/db). En utilisant ce modèle de souris diabétiques prédictif et en intégrant la composition C10 directement dans l'alimentation des rongeurs (*Chrysanthellum indicum* 1%, *Cynara scolymus* 1%, *Vaccinium myrtillus* 0,1%, *Piper nigrum* 0,001% et *Olea europaea* 0,6% de l'alimentation), cela permet de s'assurer de son efficacité « multicibles » et de son utilisation à grande échelle, les injections intraveineuses ou intrapéritonéales étant limitées à un faible nombre de personnes étant donné leur mode d'administration.

Le temps expérimental était de 7 semaines avec un « Run-in » d'1 semaine suivi de 6 semaines de complémentation avec la composition C10. Les souris males étaient âgés de 6 semaines en début de traitement. Les évaluations ont été réalisées juste avant la complémentation (t = 0), de manière hebdomadaire pour certains paramètres, et en fin de complémentation (t = 6 semaines).

Les évaluations expérimentales ont notamment porté sur :

- La mesure de la prise alimentaire (t1, t2, t3, t4 et t5 semaines) ;
- La mesure de la masse corporelle (t0, t1, t2, t3, t4, t5, t6 semaines) ;
- La mesure de la masse grasse et maigre par IRM (Imagerie par Résonance Magnétique ; t0 et t6 semaines) ;
- La mesure de la glycémie à jeun (t0, t1, t2, t3, t4, t5, t6 semaines) ;
- La mesure de l'hémoglobine glyquée (HbAlc ; t6 semaines) ;
- La sensibilité à l'insuline (test identique à celui réalisé dans les Essais A; t0 et t6 semaines) ;
- La tolérance aux glucides (test identique à celui réalisé dans les Essais A; t0 et t6 semaines) ;

- La mesure des triglycérides sériques et hépatiques (t6 semaines) ;
- La mesure du HDL cholestérol sérique (t6 semaines).

L'ensemble de ces évaluations est tout à fait connu de l'homme du métier.

Les résultats sont présentés dans le **Tableau 15** suivant. Il est important de considérer que la quantité de metformine ingérée quotidiennement par les souris était supérieure d'environ 26% à la quantité de molécules totales présentes dans la composition C10 selon l'invention. Autrement dit, la dose de metformine administrée était plus importante que la dose de molécules totales présentes dans la composition C10.

| **Paramètres** | **Contrôle** | **Metformine** | **C10** |
|---|---|---|---|
| Prise alimentaire (g/jour) | 9.6 ± 0.5 | 10.0 ± 0.6 | 10.7 ± 0.8 |
| Masse corporelle (g) | 44.7 ± 2.0 | 40.8 ± 2.1 | 36.3 ± 1.7[a] |
| Masse grasse (%) | 26.1 ± 1.5 | 23.2 ± 1.7 | 19.1 ± 1.1[a] |
| Masse maigre (%) | 17.0 ± 0.5 | 16.5 ± 0.5 | 16.5 ± 0.6 |
| Glycémie à jeun (mg/dL) | 437 ± 26 | 474 ± 24 | 160 ± 20 [a,b] |
| HbAlc (%) | 8.33 ± 0.53 | 7.59 ± 0.24 | 4.25 ± 0.20[a,b] |
| Aire sous la courbe mesurée lors d'un test oral de sensibilité à l'insuline (AUC, mg.min/dL) | 50940 ± 2968 | 32778 ± 3166 | 14109 ± 1856 [a,b] |
| Aire sous la courbe mesurée lors d'un test oral de tolérance aux glucides (AUC, mg.min/dL) | 61050 ± 2757 | 65614 ± 1577 | 28823 ± 5333 [a,b] |
| Triglycérides sériques (mg/dL) | 231.23 ± 18.29 | 256.23 ± 28.19 | 177.80 ± 19.60 [b] |
| Triglycérides hépatiques (μmol/mg tissu) | 41.10 ± 1.80 | 38.39 ± 4.84 | 21.39 ± 2.21[a,b] |
| HDL cholestérol (mg/dL) | 74.34 ± 4.10 | 99.21 ± 6.03 | 111.72 ± 8.39[a] |
| Valeurs moyennes ± SEM. Contrôle, n = 10 ; Metformine, n = 11 ; C10, n = 10. Test t de Student pour données non appariées.[a] : C10 *versus* Contrôle, $p < 0.05$.[b] : C10 *versus* Metformine, $p < 0.05$. | | | |

La **Figure 7** illustre plus particulièrement les effets de la metformine et de la composition C12 selon l'invention sur la sensibilité à l'insuline.

Les résultats illustrés dans le **Tableau 15** montrent un effet extrêmement important et surprenant de la composition C10 selon l'invention sur un ensemble de facteurs de risque du syndrome métabolique, du surpoids, de l'obésité, du diabète, de la stéatose hépatique non-alcoolique (NASH), et des maladies cardiovasculaires, à savoir :

- La diminution du poids corporel à travers une baisse de la masse grasse, sans affecter la masse maigre ;
- Une diminution de la glycémie à jeun et de l'hémoglobine glyquée ;
- Une amélioration de la tolérance aux glucides ;
- Une amélioration de la sensibilité à l'insuline ;
- Une diminution des triglycérides hépatiques et sériques ;
- Une augmentation du HDL cholestérol (« bon cholestérol »).

Non seulement la composition selon l'invention diminue tout un ensemble de facteurs de risque, mais surtout les améliore à un niveau quasiment équivalent à des souris non diabétiques (souris saines). En d'autres termes, l'invention empêche l'installation et la progression du diabète de type 2.

Les **Figures 8** et **9** illustrent les effets de la composition C10 selon l'invention sur l'évolution de la masse

corporelle et de la glycémie à jeun comparativement au groupe contrôle. La composition C10 selon l'invention limite de façon importante la prise de masse et empêche l'augmentation de la glycémie.

De plus, ses effets sont très supérieurs à ceux de la metformine, le principal médicament antidiabétique (Ferrannini E et al. Eur Heart J 2015;Jun 10 ; exemple : GLUCOPHAGE®) et démontre l'absence d'effets secondaires sur les lipides circulants, contrairement à l'OCA (obeticholic acid, un agoniste FXR destiné au traitement du diabète de type 2 et de la NASH (stéatose hépatique non-alcoolique)) développé par la société INTERCEPT qui diminue de manière drastique le HDL cholestérol (-0.2 g/L, $p < 0.01$) circulant (« bon cholestérol »). Bien au contraire, la composition selon l'invention augmente de 50.3% le taux de HDL cholestérol circulant.

Par ailleurs, l'effet de la composition selon l'invention est supérieur à celui du candidat médicament développé par la société GENFIT, le GFT 505 (Elafibranor), sur l'hémoglobine glyquée (HbAlc), le principal indicateur diagnostic du diabète de type 2 et de ses complications morbides : composition C10, HbAlc = 4% *versus* GFT 505, HbAlc = 6% pour la dose la plus haute (même modèle de souris db/db et design expérimental similaire ; Hanf R et al. Diab Vasc Dis Res 2014; 11:440-7). Une baisse de 1% supplémentaire de l'HbAlc diminue les risques d'infarctus de 14%, de maladies cardiovasculaires de 37%, et d'amputations de 43%.
De plus, la composition selon l'invention retarde, voir stoppe, la dégénérescence des cellules Béta du pancréas, cellules responsables de la sécrétion d'insuline en réponse à une augmentation de la glycémie. En effet, le diabète de type 2 induit progressivement une dégradation des cellules Béta pancréatiques liées en partie à l'hyperglycémie chronique. Cela se traduit par une augmentation de la sécrétion d'insuline dans les premiers stades de la maladie pour diminuer la glycémie (augmentation de l'insulinémie), puis par une diminution progressive de sa sécrétion (insulinémie circulante plus basse), en raison de la destruction progressive des cellules Béta (Leahy JL et al. J Clin Invest 1985;77:908-915). Les résultats obtenus soutiennent de manière forte un effet de la composition C10 sur les cellules Béta pancréatiques. En effet, après six semaines de complémentation, l'insulinémie est plus haute dans le groupe composition C10 selon l'invention *versus* Contrôle (27.40 $\pm$ 4.11 *versus* 9.64 $\pm$ 2.53 ng/mL, $p < 0.01$, respectivement), traduisant un stade d'avancé du diabète très différent : diabète avancé dans le groupe Contrôle avec destruction des cellules Béta (insulinémie basse, glycémie à jeun haute, HbAlc haute) et diabète peu avancé voire pas de diabète dans le groupe de la composition C10 selon l'invention avec des cellules Béta fonctionnelles (insulinémie haute, glycémie basse, HbAlc basse).

Ainsi, l'invention répond parfaitement au besoin de solutions de prévention et de solutions thérapeutiques nouvelles prenant en compte le caractère multifactoriel des désordres cardio-métaboliques. Cette invention représente une avancée majeure pour enrayer considérablement le développement des maladies métaboliques et leur caractère morbide.

Des mesures de biologie moléculaire au niveau hépatique ont également été réalisées. Ces évaluations ont été conduites après les six semaines de traitement et après sacrifice des animaux. En particulier, les quantités de protéines AMPK (AMP-activated protein kinase) hépatiques ont été déterminées par western blot. L'AMPK est considérée comme étant un senseur métabolique. Enzyme ubiquitaire, l'AMPK participe à la régulation coordonnée du métabolisme énergétique, de la prise alimentaire et de la sensibilité des tissus en réponse à de nombreux signaux métaboliques et hormonaux. Ces propriétés lui confèrent donc un rôle de cible pharmacologique majeure à visée métabolique (diabète, insulino-résistance, obésité) et cardiologique (ischémie cardiaque, complications liées au diabète) (Coughlan KA et al. Diabetes Metab Syndr Obes 2014;7:241-53). Augmenter les quantités d'AMPK constitue une stratégie de premier intérêt dans l'identification et la validation de traitements du diabète de type 2 et de ses complications (stéatose hépatique non-alcoolique notamment), et plus globalement pour les pathologies liées à des désordres métaboliques.

Les résultats montrent au niveau hépatique que la composition C10 selon l'invention induit une augmentation nette de la quantité d'AMPK (Contrôle n = 9, 1,00 $\pm$ 0,22 *versus* C10 n = 9, 1,96 $\pm$ 0,73, $p < 0,001$, Test U de Mann Whitney). Les résultats sont illustrés par le western blot **Figure 10.**

Le protocole expérimental réalisable par tout homme du métier est indiqué dans les lignes ci-après.

→ **AMPK**

*Extraction des protéines*

50mg de tissu congelé (foie) ont été placés dans 20 volumes de tampon NP-40 (Tris HCl 50mM, pH: 7,4, NaCl 150 mM, NaF 1mM, $Na_3VO_4$ 1mM, Nonidet P-40 1%, Sodium deoxycholate 0,25%) en présence d'1 $\mu$L de cocktail inhibiteur de protéases (P8340, Sigma Aldrich) et de tablettes inhibitrices de phosphatases (#88667 Thermo Fisher Scientific, USA). Les tissus étaient homogénéisés dans la glace au moyen d'un potter en verre puis centrifugés à 14 000 g pendant 10 minutes à 4°C avant de récupérer le surnageant. Le contenu en protéines du surnageant était dosé par kit Bio-Rad DC (Bio-Rad, USA) puis tous les échantillons étaient ramenés à la même concentration standard avant d'être dilués une seconde fois dans du tampon Laemmli 2X puis chauffés à 90°C pendant 3 minutes.

*Blotting*

Pour chaque test, une échelle de poids moléculaire et un contrôle interne étaient déposés à côté de 15 μg de chaque échantillon sur un gel à gradient en polyacrylamide (4-15% Mini-PROTEAN® TGX Stain-Free™ Gel, BioRad, USA). Les gels étaient ensuite soumis à un courant de 300V pendant 18 à 20 minutes dans un tampon d'électrophorèse (Tris 25 mM, Glycine 192 mM, SDS 0,1%) avant que les protéines ne soient transférées sur membrane PVDF grâce à un système de transfert semi-liquide (Transblot, Bio-Rad, USA) par un courant continu de 25V et 2,5A pendant 7 minutes. Les membranes étaient alors incubées dans un tampon Tween - Tris saline (TTBS : 50 mM Tris base pH: 7,5, 150 mM NaCl, and 0,01% Tween 20) enrichi avec 5% de BSA pendant 1 heure à température ambiante. Les membranes étaient ensuite rincées au TTBS puis incubées pendant la nuit à 4°C avec les anticorps anti-AMPKα (D63G4, Cell Signaling, USA). Après l'incubation, les membranes étaient à nouveau lavées au TTBS puis exposées à un anticorps secondaire anti-rabbit conjugué à de la peroxydase de raifort, à une concentration de 1:3000$^{e}$ pendant 1h à température ambiante. Les membranes étaient ensuite lavées 3 fois dans du TTBS avant d'être exposées à une solution de chimiluminescence (Clarity Western ECL; Bio-Rad, USA) pendant 1 minute. Les membranes étaient alors scannées sur un système Bio-Rad Chemidoc et l'intensité des bandes mesurées à l'aide du logiciel d'analyse d'image fourni (ImageLab V4.1, Bio-Rad, USA).

11.3 Essai C

Un des principaux inconvénients de nombreuses molécules thérapeutiques actuellement sur le marché est leur effet hypoglycémiant en aigue, avec un risque élevé d'hypoglycémie. Pour tester l'effet hypoglycémiant en aigue de la composition C10 selon l'invention, la composition a été administrée par gavage (400 mg/kg) à six souris saines C57BL/6N. La glycémie a été mesurée par biopsie au niveau de la queue avant et après gavage (t0, t15 minutes, t30 minutes). Par ailleurs, selon un protocole croisé, les mêmes souris ont reçu par gavage une solution saline avec mesures de la glycémie (t0, t15 minutes, t30 minutes). Les résultats illustrés à la **Figure 11** démontre l'absence d'effet hypoglycémiant en aigue de la composition C10 selon l'invention.

11.4 Essai D

Une composition C11 d'un extrait unique (extrait sec hydro-alcoolique) obtenu à partir de poudre de parties aériennes de *Chrysanthellum indicum,* de feuilles de *Cynara scolymus,* de fruits de *Vaccinium myrtillus,* de fruits de *Piper nigrum* et de feuilles d'*Olea europaea* (ratio 1 / 1 / 0,1 / 0,0001 / 0,6) a été testée sur le même modèle de souris diabétiques utilisé dans les essais A et B.
Le temps expérimental était de 6 semaines avec un « Run-in » d'1 semaine suivi de 5 semaines de complémentation avec la composition C11. Les souris males étaient âgés de 6 semaines en début de traitement. L'évaluation a porté principalement sur l'évolution de la masse corporelle. Les mesures ont été réalisées juste avant la complémentation (t = 0), de manière hebdomadaire pour certains paramètres, et en fin de complémentation (t = 5 semaines). La composition a été intégrée dans l'alimentation des rongeurs à raison de 2,7% de la nourriture (même quantité que la composition C10).
La **Figure 12** illustre les effets de la composition C11 selon l'invention sur l'évolution de la masse corporelle comparativement au groupe contrôle. La composition C11 selon l'invention limite fortement la prise de masse corporelle.

III. EVALUATION IN VITRO DE l'EFFICACITE DE LA COMPOSITION

Des expérimentations *in vitro* ont également été réalisées pour démontrer les effets des compositions C10 et C11 selon l'invention. La composition C12 d'association de molécules selon l'invention a également été testée : acide dicaféoylquinique + apigenine-7-O-glucuronide + acide monocaféoylquinique + oleuropéine + pipérine (ratio 1 / 1 / 1 / 1 / 0.001).
Différents tests d'activité enzymatique ont été effectués afin de déterminer si les compositions selon l'invention inhibaient des enzymes stratégiques du métabolisme des glucides et des lipides : l'alpha-glucosidase, DDP-IV et l'HMG-CoA réductase. Une action sur l'ensemble de ces cibles stratégiques permet une prise en charge globale du diabète de type 2 et de ses complications (pied diabétique, rétinopathie, perte de la vision, néphropathie, évènements cardiovasculaires, stéatose hépatique non-alcoolique ou NASH).

→ HMG-CoA réductase

Pour le contrôle des lipides circulants, l'objectif est de réduire la concentration de cholestérol sérique en ciblant principalement l'inhibition de la biosynthèse du cholestérol (inhibition de la 3-Hydroxy-3-Methyl-Glutaryl-Coenzyme A

ou HMG-CoA réductase). La pravastatine est l'une des molécules référentes pour la diminution du LDL cholestérol et du cholestérol total. Elle est notamment commercialisée sous le nom d'ELISOR®, PRAVASTATINE MYLAN, VASTEN®, PRAVASTATINE TEVA.

→ Alpha-glucosidase

L'alpha-glucosidase est une enzyme qui catalyse l'étape finale de processus de digestion des hydrates de carbone. Les inhibiteurs de l'alpha-glucosidase (exemples de médicaments entrant dans cette catégorie : GLUCOR®, DIASTABOL®) sont utilisés pour diminuer la glycémie postprandiale, considérée comme un facteur de risque indépendant des complications macro-vasculaires dans le diabète (Kim JS et al. Biosci Biotechnol Biochem 2000; 64 :2458-61).

→ DPP-IV (dipeptidyl peptidase-IV)

Les inhibiteurs de DPP-IV sont considérés comme une des stratégies de prise en charge du diabète de type 2 les plus prometteuses (von Geldern TW et al. Drug Development Research 2006; 67:627-42). DPP-IV inactive rapidement les hormones GLP-1 (glucagon-like peptide 1) et GIP (glucose-dependent insulinotropic polypeptide), également connues sous le nom d'incrétines. L'inhibition de DPP-IV permet d'augmenter notamment l'action de GLP-1 et de GIP permettant ainsi une stimulation physiologique de la sécrétion d'insuline et une inhibition de la sécrétion de glucagon par le pancréas, et par voie de conséquence une diminution de la glycémie. Par ailleurs, l'inhibition de DPP-IV favorise la vidange gastrique et provoque un effet anorexigène central. L'inhibition de DPP-IV permet donc une meilleure régulation des glucides chez les personnes diabétiques. Les études cliniques montrent par ailleurs que les inhibiteurs de DPP-IV présentent une bonne efficacité avec une bonne tolérabilité dans la prise en charge de l'hyperglycémie chez les personnes atteintes de diabète de type 2, sans gain de poids et de phénomènes d'hypoglycémie (Green BD et al. Diabetes Vasc Dis Res 2006; 3:159-65). Un des inhibiteurs de référence est la diprotine A.

Le protocole expérimental pour évaluer le potentiel inhibiteur des compositions C10, C11 et C12 étaient les suivants :

*Préparation des extraits, mélanges d'extraits et mélange de molécules pures de synthèse*

Pour les tests d'inhibition de l'HMG-CoA réductase et des glucosidases, les extraits végétaux étaient dilués dans des mélanges eau ultra pure/éthanol, dont les proportions variaient selon le type d'extrait, afin d'obtenir des solutions homogènes.

Après homogénéisation, les échantillons étaient centrifugés à 4000 rpm pendant 10 minutes et les surnageants étaient récupérés (centrifugeuse 1-15 Fisher Bioblock Scientif, Sigma Aldrich®). Les molécules étaient diluées soit en eau ultra pure/DMSO 90/10 (v/v), soit en eau ultra pure/éthanol 50/50 (v/v).

Les mélanges de plusieurs extraits végétaux étaient dilués en eau/éthanol 50/50 (v/v), puis étaient passés aux ultrasons pendant 15 minutes, afin d'homogénéiser le milieu (utilisation d'une sonde à ultrasons UP50H Hielscher® à puissance maximale). Enfin, ils étaient centrifugés à 4000 rpm pendant 10 minutes et les surnageants étaient récupérés (centrifugeuse 1-15 Fisher Bioblock Scientif, Sigma Aldrich®).

Dans le cas du test DPP-IV, les échantillons étaient dilués soit en tampon Trisma-HCl (pH8,0 ; 100 mM), soit en eau ultra pure/DMSO 90/10 (v/v).

*Test d'inhibition de l'HMG-CoA réductase*

Ce test permet de mesurer, par une diminution d'absorbance au cours du temps, à $\lambda$=340nm, l'efficacité de l'inhibition de l'activité enzymatique. Le mécanisme de réaction est le suivant :

$$\text{HMG-CoA} + 2\text{NADPH} + 2\text{H}^+ \rightarrow \text{mévalonate} + 2\text{NADP}^+ + \text{CoA-SH}$$

La disparition du substrat NADPH (absorbant à 340 nm) est donc suivie au cours du temps. Une diminution de la variation de l'absorbance à 340 nm au cours du temps par rapport à la réaction contrôle sans inhibiteur (100% activité) témoigne d'une inhibition de l'enzyme. Dans un premier temps il faut préparer les solutions à partir du kit Sigma® :

- le tampon 5X fourni doit être dilué à une concentration de 1X en eau ultra pure ;
- le NADPH est solubilisé par l'ajout de tampon 1X, puis réparti en tube de 100$\mu$L ;
- la solution de départ d'enzyme est répartie en aliquote de 25$\mu$L.

Le tampon et le NADPH étaient conservés à -20°C et les aliquotes d'enzymes étaient stockés à -80°C jusqu'à utilisation.

Les manipulations étaient réalisées directement en microplaque 96 puits (NUNC96 ou SARSTEDT96). Les tampons, substrats, inhibiteurs et l'enzyme étaient ajoutés dans un ordre spécifique (voir ci-dessous). Pour chaque microplaque il était nécessaire de préparer un blanc, un contrôle et différents essais ont été réalisés. Chaque élément était réalisé en double (voir en triple) :

- réalisation du blanc : 24 μL du solvant utilisé pour les échantillons, 12 μL d'HMG-CoA, 158 μL de tampon 1X, 4 μL de NADPH ; incubation à 37°C, 3 à 5 min ;
- réalisation du contrôle négatif : 24 μL du solvant utilisé pour les échantillons, 12 μL d'HMG-CoA, 158 μL de tampon 1X, 4 μL de NADPH ; incubation à 37°C, 3 à 5 min ; ajout 2 μL d'enzyme ;
- réalisation de l'essai : 24 μL de l'échantillon potentiellement inhibiteur, 12 μL d'HMG-CoA, 158 μL de tampon 1X, 4μL de NADPH ; incubation à 37°C, 3 à 5 min ; ajout 2 μL d'enzyme.

Après préparation de la plaque, le suivi cinétique était effectué dans un lecteur de microplaques (BMG Labtech) sur 16 minutes, 25 secondes, avec des valeurs prises toutes les 25 secondes à λ=340nm, soit un total de 40 cycles. A partir de cela les pourcentages d'inhibition pouvaient être calculés.

*Test d'inhibition des α-glucosidoses*

Les tests d'inhibition des α-glucosidases étaient réalisés en suivant par spectrophotométrie, à λ=405 nm, la formation du produit para-nitrophénol (PNP) de couleur jaune à partir du substrat synthétique : le para--nitrophényl-α-D-glucopyranoside (PNPg), selon la réaction d'hydrolyse suivante, catalysée par les α-glucosidases :

$$\text{PNPg} + H_2O \rightarrow \text{PNP} + \text{glucose}$$

Une diminution de la variation de l'absorbance, à λ=405 nm, au cours du temps par rapport à la réaction contrôle sans inhibiteur (100% activité) témoigne d'une inhibition de l'enzyme. L'enzyme humaine recombinante produite dans *Saccharomyces cerevisiae* (maltase) (Sigma Aldrich®, G0660) était diluée dans du tampon phosphate (0,1 M, pH 6,8) pour passer d'une solution mère théoriquement à 120 U/mL, à une solution avec une activité de 1,6 U/mL, utilisée pour les tests.

Lors des tests d'inhibition des α-glucosidases, les manipulations étaient réalisées directement en microplaque 96 puits (NUNC96 ou SARSTEDT96). Les tampons, substrats, inhibiteurs et l'enzyme sont ajoutés dans un ordre spécifique (voir ci-dessous). Pour chaque microplaque préparée, un blanc, un contrôle et différents essais sont réalisés. Chaque élément est réalisé en double (voir en triple) :

- réalisation du blanc : 100 μL de tampon phosphate (0,1 M, pH 6,8), 20 μL du solvant utilisé pour les échantillons, incubation à 37°C, 10 à 15 min, puis ajout de 20 μL de PNPg à 2,5 mM (solubilisé dans tampon phosphate) ;
- réalisation du contrôle négatif : 100 μL de tampon phosphate (0,1 M, pH 6,8), 20 μL du solvant utilisé pour les échantillons, 20 μL d'enzymes à 1,6 U/mL, incubation à 37°C, 10 à 15 min, puis ajout de 20 μL de PNPg à 2,5 mM (solubilisé dans tampon phosphate);
- réalisation de l'essai : 100 μL de tampon phosphate (0,1 M, pH 6,8), 20μL de l'échantillon potentiellement inhibiteur, 20 μL d'enzyme à 1,6 U/mL, incubation à 37°C, 10 à 15 min, puis ajout de 20 μL de PNPg à 2,5mM (solubilisé dans tampon phosphate).

Après préparation de la plaque, le suivi cinétique était effectué dans un lecteur de microplaques (BMG Labtech) sur 30 minutes, avec des valeurs prises toutes les 2 minutes à λ=405nm, soit un total de 16 cycles. A partir de cela les pourcentages d'inhibition pouvaient être calculés.

*Test d'inhibition de la DPP-IV*

Les tests d'inhibition de la DPP-IV sont réalisés en suivant par spectrophotométrie, à 385 nm, la formation du produit p-nitroaniline à partir du substrat synthétique : le Gly-L-Pro-p-Nitroanilide, selon la réaction d'hydrolyse suivante catalysée par la DPP-IV :

$$\text{Gly-L-Pro-p-Nitroanilide} + H_2O \rightarrow \text{p-nitroaniline} + \text{Gly-L-pro}$$

Une diminution de la variation de l'absorbance à 385 nm au cours du temps par rapport à la réaction contrôle

sans inhibiteur (100% activité) témoigne d'une inhibition de l'enzyme. L'enzyme utilisée était à une concentration de 0,045 U/mL (avec U=μmol/min), il fallait donc la diluer. La dilution étant grande, il était nécessaire de réaliser deux dilutions successives.

Première dilution (obtention d'une solution à $5.10^{-4}$ U/mL) : $D1 = \frac{5.10^{-4} * 450}{0,045} = 5\ \mu L$ (Vfinal : 450 μL ; V(Solution mère d'enzyme)= 5 μL)

Seconde dilution (obtention d'une solution à $5.10^{-6}$ U/mL) $D2 = \frac{5.10^{-6} * 1000}{5.10^{-4}} = 10\ \mu L$ (Vfinal : 1000 μL ; V(D1)=10 μL)→ la solution D2 était la solution utilisable.

Les manipulations étaient réalisées directement en microplaque 96 puits (NUNC96 ou SARSTEDT96). Les tampons, substrats, inhibiteurs et l'enzyme étaient ajoutés dans un ordre spécifique (voir ci-dessous). Pour chaque microplaque préparée, un blanc, un contrôle et différents essais étaient réalisés. Chaque élément était réalisé en double (voir en triple) :

- réalisation du blanc : 25 μL de l'échantillon, 25 μL de substrat (Gly-L-pro-p-nitroanilide solubilisé en tampon Tris-HCl (100 mM, pH8,0) à une concentration de 1,6 mM) ;
- réalisation du contrôle négatif : 25 μL du diluant utilisé pour l'échantillon, 25 μL de substrat, incubation à 37°C 10 min, 50 μL d'enzyme à $5.10^{-6}$ U/mL (préparée en tampon TrisHCl 100 mM, pH8,0) ;
- réalisation de l'essai : 25μL d'échantillon potentiellement inhibiteur 25μL de substrat, incubation à 37°C, 10 min, 50 μL d'enzyme à $5.10^{-6}$ U/mL;

Après préparation de la plaque, le suivi cinétique était effectué dans un lecteur de microplaques (BMG Labtech) sur 30 minutes, avec des valeurs prises toutes les minutes à λ=385 nm, soit un total de 31 cycles. A partir de cela les pourcentages d'inhibition pouvaient être calculés.

*Calculs des pourcentages inhibitions*

De manière générale, quel que soit le test, pour une concentration donnée d'un inhibiteur, une courbe d'équation : absorbance=f(temps) (en minutes) était réalisée. De même, des contrôles (sans inhibiteur) et des blancs (sans enzymes) étaient réalisés systématiquement. Au niveau des différentes courbes obtenues, seules les parties linéaires en début de cinétique étaient retenues, car elles correspondaient aux conditions permettant de déterminer les vitesses initiales. Ainsi, la pente en absorbance/minute était proportionnelle à la vitesse d'apparition du produit (glucosidase et DPP-IV) ou de disparition du substrat (HMG-CoA réductase, pente négative).

Selon les types de tests, les pourcentages d'inhibition des différentes enzymes ont été calculés par différentes méthodes :

- pour les tests d'inhibition de l'HMG-CoA réductase et des α-glucosidases la formule de calcul est identique : $$\%\ \text{inhibition} = \frac{\text{pente contrôle} - \text{pente échantillon}}{\text{pente contrôle}} * 100.$$
- pour le test d'inhibition du DPP4, la formule est légèrement différente car des blancs sont pris en compte :

$$\%\ \text{inhibition} = \frac{(\text{pente contrôle} - \text{pente blanc}) - (\text{pente échantillon} - \text{pente blanc})}{(\text{pente contrôle} - \text{pente blanc})} * 100.$$

Au final, une valeur moyenne de pourcentage d'inhibition était calculée pour chaque concentration d'inhibiteur, ainsi que l'écart standard de chaque valeur. Si les valeurs d'écart standard étaient supérieures à 10%, de nouveaux tests en duplicata étaient réalisés, jusqu'à obtenir des valeurs inférieures à 10%.

Les résultats obtenus sur les trois enzymes pour les compositions C10 et C11 selon l'invention sont résumés dans le **Tableau 16** ci-dessous.

| Produit | CI 50[a] (μg/mL en puit) | % d'inhibition | |
|---|---|---|---|
| | **Alpha-glucosidase** (Human recombinant *Saccharomyces Cerevisiae)* | **DPP-IV** [b] (DPP-IV human recombinant expressed in SF9 cells) | **HMG-CoA reductase**[c] (Catalytic domain of the human enzyme - recombinant GST fusion protein expressed in E. Coli) |
| Acarbose | 86,3 | Absence d'inhibition | Absence d'inhibition |
| Diprotine A | Absence d'inhibition | 100% | Absence d'inhibition |
| Pravastatine | Absence d'inhibition | Absence d'inhibition | 100% |
| Composition C10 | 4,3 | 15,3% | 48,5% |
| Composition C11 | 4,9 | 29,3% | 32,5% |
| [a] CI 50 : concentration d'inhibiteur pour laquelle l'enzyme n'a plus que 50% de son activité. [b] Concentration d'échantillon fixée à 1,25mg/mL. [c] Concentration d'échantillon fixée à 60μg/mL. | | | |

Les résultats obtenus sur les trois enzymes pour l'association de molécules (composition C12 selon l'invention) sont résumés dans le **Tableau 17** ci-dessous.

| Produit | CI 50[a] (μg/mL en puit) | % d'inhibition | |
|---|---|---|---|
| | **Alpha-glucosidase** (Human recombinant *Saccharomyces Cerevisiae)* | **DPP-IV** [b] (DPP-IV human recombinant expressed in SF9 cells) | **HMG-CoA reductase**[c] (Catalytic domain of the human enzyme - recombinant GST fusion protein expressed in E. Coli) |
| Acarbose | 86.3 | Absence d'inhibition | Absence d'inhibition |
| Diprotine A | Absence d'inhibition | 89.3% | Absence d'inhibition |
| Pravastatine | Absence d'inhibition | Absence d'inhibition | 100% |
| Composition C12 | 19.7 | 10.3% | 30.1% |
| [a] CI 50 : concentration d'inhibiteur pour laquelle l'enzyme n'a plus que 50% de son activité. [b] Concentration d'échantillon fixée à 250 μg/mL. [c] Concentration d'échantillon fixée à 60 μg/mL. | | | |

On constate que contrairement aux molécules inhibitrices référentes de chaque enzyme [acarbose (médicament), diprotine A (inhibiteur de référence), pravastatine (médicament) respectivement], les compositions C10, C11 et C12 selon l'invention sont de façon surprenante les seules à inhiber les 3 enzymes.

En agissant de manière simultanée sur plusieurs processus de régulation, les compositions selon l'invention représentent un moyen de prévention et un moyen thérapeutique avantageux pour prévenir et traiter le diabète, les dyslipidémies et leurs complications. Les stratégies thérapeutiques actuelles consistent à associer plusieurs médicaments afin de réduire les différents facteurs de risque individuellement. Néanmoins, la combinaison de drogues peut parfois engendrer des réactions secondaires graves comme par exemple, l'administration simultanée de fibrates et de statines qui augmente le risque de myopathie (Denke MA J Manag Care Pharm 2003; 9:17-9). Il existe donc aujourd'hui un réel besoin de solutions de prévention et de médicaments dont le mécanisme d'action « multicibles » présente des avantages en termes de compliance, de tolérance et d'efficacité. Ainsi les compositions selon l'invention permettent de diminuer le risque de maladies cardiovasculaires et de prévenir et traiter chaque dysfonctionnement et/ou ses conséquences pris indépendamment.

## Revendications

1. Composition comprenant au moins un mélange de molécules constitué au moins :

- par un extrait unique obtenu à partir d'au moins *Chrysanthellum indicum, Cynara scolymus* et *Vaccinium myrtillus,* d'*Olea europaea* et *Piper,* et/ou
- par un extrait unique obtenu à partir d'au moins *Chrysanthellum indicum, Cynara scolymus,* et *Vaccinium myrtillus* et d'*Olea europaea* et par de la pipérine synthétique,

ledit mélange de molécules comprenant :

- au moins une molécule choisie parmi l'apigenine-7-O-glucuronide, la chrysanthelline A, la chrysanthelline B, l'acide caféique, la lutéoline, la maritimétine, l'ériodictyol, l'isookanine, l'apigénine, la lutéoline-7-O-glucoside, la maritiméine, la maréine, l'ériodictyol-7-O-glucoside, la flavomaréine, l'apigénine-8-C-α-L-arabinoside-6-C-β-D-glucoside (shaftoside), l'apigénine-6,8-C-di-β-D-glucopyranoside (vicénine-2), et
- au moins une molécule choisie parmi un acide dicaféoylquinique, un acide sulfo-monocaféoylquinique, la lutéoline, la lutéoline-7-0-glucoside, la lutéoline-7-0-glucuronide, l'apigenine-7-O-glucoside, la cynaropicrine, et
- au moins une molécule choisie parmi un acide monocaféoylquinique, la delphinidine-3-galactoside, la delphinidine-3-glucoside, la cyanidine-3-galactoside, la delphinidine-3-arabinoside, la cyanidine-3-glucoside, la petunidine-3-galactoside, la cyanidine-3-arabinoside, la petunidine-3-glucoside, la péonidine-3-galactoside, la petunidine-3-arabinoside, la péonidine-3-glucoside, la malvidine-3-galactoside, la malvidine-3-glucoside, la malvidine-3-arabinoside,
- au moins une molécule choisie parmi l'oleuropéine et l'hydroxytyrosol, et
- au moins de la pipérine.

2. Composition selon l'une des précédentes revendications, **caractérisée** en ce *Piper* est choisi parmi *Piper nigrum, Piper aduncum* et/ou *Piper longum.*

3. Composition selon l'une des précédentes revendications **caractérisée en ce que** le ou les extrait(s) unique(s) est(sont) obtenu(s) à partir de :

- plante entière et/ou des parties aériennes de *Chrysanthellum indicum,*
- plante entière et/ou de feuilles de *Cynara scolymus,*
- plante entière et/ou de fruits de *Vaccinum myrtillus.*

4. Composition selon l'une des précédentes revendications, **caractérisée en ce qu'**elle présente au moins une des caractéristiques suivantes :

- elle comprend une quantité d'extrait de *Chrysanthellum indicum* permettant l'administration d'au moins 0,00001g d'extrait de *Chrysanthellum* indicum par kg de poids corporel de la personne à laquelle la composition est administrée et par jour, et/ou
- elle comprend une quantité d'extrait de *Cynara scolymus* permettant l'administration d'au moins 0,00001g d'extrait de *Cynara scolymus* par kg de poids corporel de la personne à laquelle la composition est administrée et par jour, et/ou
- elle comprend une quantité d'extrait de *Vaccinium myrtillus* permettant l'administration d'au moins 0,00001g d'extrait de *Vaccinium myrtillus* par kg de poids corporel et par jour, et/ou
- elle comprend une quantité de pipérine permettant l'administration d'au moins 0,001mg de pipérine par kg de poids corporel de la personne à laquelle la composition est administrée et par jour,
- elle comprend une quantité d'extrait unique correspondant à une administration d'au moins 0,00001g d'extrait unique par kg de poids corporel de la personne à laquelle la composition est administrée et par jour.

5. Composition selon l'une des précédentes revendications, **caractérisée en ce que** le mélange comprend au moins un acide dicaféoylquinique, l'apigenine-7-O-glucuronide, un acide monocaféoylquinique, de la pipérine et l'oleuropéine.

6. Composition selon l'une des précédentes revendications, **caractérisée en ce qu'**elle comprend également au moins un élément supplémentaire ajouté en plus du mélange de molécules, ledit élément supplémentaire étant choisi parmi :

- les vitamines suivantes : B1, B2, B3, B5, B6, B8, B9, B12 C, A, D, E, K1 et K2 ;
- les composés suivants : acide obeticholique, acide corosolique, acides gras polyinsaturés de la famille des oméga 6 et/ou oméga 3, acide orotique, acide pangamique, acide para-amino-benzoïque, amygdaline, béta-glucanes, carnitine, diméthylglycine, imeglimine, isoflavones, L-arginine, oxytocine, pectine, pyridoxamine, resvératrol, viniférine, L-citrulline ;
- les oligo-éléments et minéraux suivants : arsenic, bore, calcium, cuivre, fer, fluor, iode, lithium, manganèse, magnésium, molybdène, nickel, phosphore, sélénium, vanadium, zinc ;
- les microconstituants à caractère non indispensable suivants : acide linolénique conjugué, acide lipoïque, caroténoïdes, carnitine, choline, coenzyme Q10, phytostérols, polyphénols de la famille des tannins et des lignanes, taurine ;
- les fructo-oligosaccharides, les galacto-oligosaccharides ;
- les ferments lactiques ;
- les levures ;
- les champignons;
- les produits dérivés d'insectes compatibles avec le secteur alimentaire et pharmaceutique ;
- la marijuana et le haschisch ;
- des agents d'enrobage;
- des arômes;
- des acidifiants ;
- des antiagglomérants ;
- des épaississants ;
- des stabilisants ;
- des émulsifiants ;
- des agents de charge ;
- des excipients.

**7.** Composition selon l'une des précédentes revendications, **caractérisée en ce qu'**elle se présente sous forme de poudre, de gel, d'émulsion, ou sous forme liquide.

**8.** Composition selon l'une des précédentes revendications **caractérisée en ce qu'**elle se présente sous forme de comprimés, capsules, gélules, sticks, sachets, ampoules, compte-gouttes ou sous forme injectable.

**9.** Composition selon l'une des précédentes revendications, pour son utilisation comme médicament.

**10.** Composition selon l'une des revendications 1 à 8, pour son utilisation comme médicament ou produit de nutrition dans la prévention et/ou la lutte contre les dérèglements pathologiques du métabolisme glucidique et/ou lipidique chez l'Homme ou l'animal.

**11.** Composition pour son utilisation selon la revendication 9 ou 10, dans la prévention et/ou la lutte contre les diabètes de type 1 et 2 et/ou des maladies du foie gras non alcoolique et/ou des pathologies cardiovasculaires et/ou des pathologies liées à une insulino-résistance.

**12.** Composition pour son utilisation selon la revendication 11, **caractérisée en ce que** la maladie du foie gras non alcoolique est la stéatose hépatique non alcoolique.

**13.** Composition pour son utilisation selon la revendication 11, **caractérisée en ce que** les pathologies cardiovasculaires sont les cardiopathies coronariennes, les maladies cérébro-vasculaires, les artériopathies périphériques, les thromboses veineuses profondes.

**14.** Composition pour son utilisation selon la revendication 11, **caractérisée en ce que** la pathologie liée à une insulino-résistance est la maladie d'Alzheimer.

**15.** Composition pour son utilisation selon l'une des revendications 11 à 14, **caractérisée** en qu'elle est utilisée en combinaison avec au moins un agent thérapeutique antidiabétique choisi parmi les biguanides dont la metformine, les inhibiteurs de la dipeptidyl peptidase-IV (DPP-IV), les analogues au glucagon-like peptide-1 (GLP-1), les thiazolidinediones (TZDs), les sulfonylurées, les insulines rapides et lentes, les inhibiteurs du sodium glucose cotransporter-2 (SGLT2), les inhibiteurs des glycosidases (acarbose, miglitol, voglibose, peptides contenant la séquence alanine-proline ou proline-alanine), les molécules de la famille du fibranor comme l'elafibranor, ou les molécules ciblant les récepteurs nucléaires et notamment les récepteurs RORs ($\alpha$, $\beta$, $\gamma$) et Rev-Erbs ($\alpha$, $\beta$).

**16.** Composition pour son utilisation selon la revendication 9 ou 10, dans la prévention et/ou la lutte contre la dyslipidémie.

**17.** Composition pour son utilisation selon la précédente revendication, **caractérisée** en qu'elle est utilisée en combinaison avec un agent thérapeutique hypolipémiant choisi parmi : les statines, les fibrates, l'acide nicotinique, les résines échangeuses d'ions, les inhibiteurs de l'absorption du cholestérol, les acides gras polyinsaturés oméga 3, le tiadénol, et les agonistes du récepteur nucléaire FXR (Farnesoid X Receptor).

**18.** Composition pour son utilisation selon la revendication 9 ou 10, dans la prévention et/ou la lutte contre l'obésité et le surpoids et/ou le syndrome métabolique et/ou les problèmes pathologiques de tension artérielle.

## Patentansprüche

**1.** Zusammensetzung mit wenigstens einer Molekülmischung, die wenigstens zusammengesetzt ist aus:

- einem Einzelextrakt, der auf der Grundlage von wenigstens *Chrysanthellum indicum, Cynara scolymus,* und *Vaccinium myrtillus, Olea europaea* und aus *Piper* hergestellt ist und/oder
- einem Einzelextrakt, der auf der Grundlage von wenigstens *Chrysanthellum indicum, Cynara scolymus, Vaccinium myrtillus,* und *Olea europaea* und aus synthetischem Piperin hergestellt ist,

wobei die Molekülmischung umfasst:

- wenigstens ein Molekül, das aus Apigenin-7-O-glucuronid, Chrysanthellin A, Chrysanthellin B, Kaffeesäure, Luteolin, Maritimetin, Eriodictyol, Isookanin, Apigenin, Luteolin-7-O-glucosid, Maritimein, Marein, Eriodictyol-7-O-glucosid, Flavomarein, Apigenin-8-C-α-L-arabinosid-6-C-β-D-glucosid (Shaftosid), Apigenin-6,8-C-di-β-D-glucopyranosid (Vicenin-2) ausgewählt ist, und
- wenigstens ein Molekül, das aus Dicaffeoylchinasäure, Sulfomonocaffeoylchinasäure, Luteolin, Luteolin-7-O-glucosid, Luteolin-7-O-glucuronid, Apigenin-7-O-glucosid, Cynaropicrin ausgewählt ist, und
- wenigstens ein Molekül, das aus Monocaffeoylchinasäure, Delphinidin-3-galactosid, Delphinidin-3-glucosid, Cyanidin-3-galactosid, Delphinidin-3-arabinosid, Cyanidin-3-glucosid, Petunidin-3-galactosid, Cyanidin-3-arabinosid, Petunidin-3-glucosid, Peonidin-3-galactosid, Petunidin-3-arabinosid, Peonidin-3-glucosid, Malvidin-3-galactosid, Malvidin-3-glucosid, Malvidin-3-arabinosid ausgewählt ist,
- wenigstens ein Molekül, das aus Oleuropein und Hydroxytyrosol ausgewählt ist, und
- wenigstens Piperin.

**2.** Zusammensetzung nach einem der vorhergehenden Ansprüche, **dadurch gekennzeichnet, dass** *Piper* aus *Piper nigrum, Piper aduncum* und/oder *Piper longum* ausgewählt ist.

**3.** Zusammensetzung nach einem der vorhergehenden Ansprüche, **dadurch gekennzeichnet, dass** der/die Einzelextrakt(e) hergestellt sind auf der Grundlage von:

- der ganzen Pflanze und/oder den oberirdischen Teilen von *Chrysanthellum indicum,*
- der ganzen Pflanze und/oder aus den Blättern von *Cynara scolymus,*
- der ganzen Pflanze und/oder aus den Früchten von *Vaccinium myrtillus.*

**4.** Zusammensetzung nach einem der vorhergehenden Ansprüche, **dadurch gekennzeichnet, dass** diese wenigstens eine der folgenden Eigenschaften aufweist:

- diese weist eine Menge an Extrakt aus *Chrysanthellum indicum* auf, die die tägliche Anwendung von wenigstens 0,00001 g Extrakt aus *Chrysanthellum indicum* pro kg Körpergewicht der Person gestattet, der die Zusammensetzung verabreicht wird, und/oder
- diese weist eine Menge an Extrakt aus *Cynara scolymus* auf, die die tägliche Anwendung von wenigstens 0,00001 g Extrakt aus *Cynara scolymus* pro kg Körpergewicht der Person gestattet, der die Zusammensetzung verabreicht wird, und/oder
- diese weist eine Menge an Extrakt aus *Vaccinium myrtillus* auf, die die tägliche Anwendung von wenigstens 0,00001 g Extrakt aus *Vaccinium myrtillus* pro kg Körpergewicht gestattet, und/oder
- diese weist eine Menge an Piperin auf, die die tägliche Anwendung von wenigstens 0,001 mg Piperin pro kg Körpergewicht der Person gestattet, der die Zusammensetzung verabreicht wird,
- diese weist eine Menge an Einzelextrakt auf, die der täglichen Anwendung von wenigstens 0,00001 mg Einzelextrakt pro kg Körpergewicht der Person entspricht, der die Zusammensetzung verabreicht wird.

**5.** Zusammensetzung nach einem der vorhergehenden Ansprüche, **dadurch gekennzeichnet, dass** die Mischung wenigstens Dicaffeoylchinasäure, Apigenin-7-O-glucuronid Monocaffeoylchinasäure, Piperin und Oleuropein aufweist.

**6.** Zusammensetzung nach einem der vorhergehenden Ansprüche, **dadurch gekennzeichnet, dass** diese wenigstens ein Zusatzelement aufweist, das der Molekülmischung hinzugesetzt ist, wobei das Zusatzelement ausgewählt ist aus:

- den folgenden Vitaminen: B1, B2, B3, B5, B6, B8, B9, B12 C, A, D, E, K1 und K2;
- den folgenden Verbindungen: Obeticholsäure, Corosolsäure, den mehrfach ungesättigten Fettsäuren aus der Familie der Omega-6- und/oder Omega-3-Säuren, Orotsäure, Pangamsäure, Para-Amino-Benzoesäure, Amygdalin, Beta-Glucan, Carnitin, Dimethylglycin, Imeglimin, Isoflavon, L-Arginin, Oxytocin, Pektin, Pyridoxamin, Resveratrol, Viniferin, L-Citrullin;
- den folgenden Spurenelementen und Mineralien: Arsen, Bor, Calcium, Kupfer, Eisen, Fluor, Iod, Lithium, Mangan, Magnesium, Molybdän, Nickel, Phosphor, Selen, Vanadium, Zink;
- den folgenden nicht lebensnotwendigen Mikrokomponenten: konjugierte Linolsäure, Liponsäure, Karotenoide, Carnitin, Cholin, Coenzym Q10, Phytosterole, Polyphenole aus der Familie der Tannine und Lignane, Taurin;
- den Fructo-Oligosacchariden, den Galacto-Oligosacchariden;
- den Milchsäuregärungserregern;
- den Hefen;
- den Pilzen;
- den von Insekten abgeleiteten Produkten, die mit dem Lebensmittelsektor und Heilmittelsektor kompatibel sind;
- dem Marihuana und Haschisch;
- den Überzugsmitteln;
- den Aromen;
- den Ansäuerungsmitteln;
- den Antiklumpmitteln;
- den Verdickungsmitteln;
- den Stabilisatoren;
- den Emulgatoren;
- den Füllstoffen;
- den Hilfsstoffen.

**7.** Zusammensetzung nach einem der vorhergehenden Ansprüche, **dadurch gekennzeichnet, dass** diese in der Form von Pulver, eines Gels, einer Emulsion oder in flüssiger Form vorliegen.

**8.** Zusammensetzung nach einem der vorhergehenden Ansprüche, **dadurch gekennzeichnet, dass** diese in der Form von Tabletten, Kapseln, Stiften, Beuteln, Ampullen, Tropfbehältern oder in einer injizierbaren Form vorliegen.

**9.** Zusammensetzung nach einem der vorhergehenden Ansprüche, zu deren Verwendung als Medikament.

**10.** Zusammensetzung nach einem der Ansprüche 1 bis 8, zu deren Verwendung als Medikament oder Nährprodukt bei der Prävention und/oder der Bekämpfung von pathologischen Störungen des Zucker- und/oder Fettstoffwechsels beim Menschen oder beim Tier.

**11.** Zusammensetzung zu deren Verwendung nach Anspruch 9 oder 10, bei der Prävention und/oder der Bekämpfung von Diabetes vom Typ 1 oder 2 und/oder von Krankheiten der nicht-alkoholischen Fettleber und/oder kardiovaskulären Pathologien und/oder Pathologien, die mit einer Insulinresistenz verknüpft sind.

**12.** Zusammensetzung zu deren Verwendung nach Anspruch 11, **dadurch gekennzeichnet, dass** die Erkrankung der nicht-alkoholischen Fettleber die nicht-alkoholische Steatosis hepatis ist.

**13.** Zusammensetzung zu deren Verwendung nach Anspruch 11, **dadurch gekennzeichnet, dass** die kardiovaskulären Pathologien koronale Herzkrankheiten, zerebrovaskuläre Krankheiten, periphäre Arteriopathien oder Thrombosen der tiefen Venen sind.

**14.** Zusammensetzung zu deren Verwendung nach Anspruch 11, **dadurch gekennzeichnet, dass** die mit der Insulinresistenz verknüpfte Pathologie die Alzheimerkrankheit ist.

**15.** Zusammensetzung zu deren Verwendung nach einem der Ansprüche 11 bis 14, **dadurch gekennzeichnet, dass** diese in Kombination mit wenigstens einem antidiabetischen Heilmittel verwendet wird, das unter den Biguaniden, darunter Metformin, den Inhibitoren von Dipeptidylpeptidase-IV(DPP-IV), den Analogen zu Glucagon-like Peptide-1 (GLP-1), den Thiazolidindionen (TZD), den Sulfonylharnstoffen, den schnellen und langsamen Insulinen, den Inhibitoren des Natrium/Glukose-Cotransporters-2 (SGLT2), den Inhibitoren der Glycosidasen (Acarbose, Miglitol, Voglibose, Peptiden mit der Folge Alanin-Prolin oder Prolin-Alanin), den Molekülen der Familie von Fibranor, wie Elafibranor, oder den Molekülen ausgewählt ist, die auf die Kernrezeptoren und insbesondere auf die Rezeptoren ROR (α, β, γ) und Rev-Erbs (α, β) abzielen.

**16.** Zusammensetzung zu deren Verwendung nach dem Anspruch 9 oder 10, bei der Prävention und/oder der Bekämpfung von Fettstoffwechselstörungen.

**17.** Zusammensetzung zu deren Verwendung nach dem vorhergehenden Anspruch, **dadurch gekennzeichnet, dass** diese in Kombination mit einem lipidsenkenden Heilmittel verwendet wird, das ausgewählt ist unter: den Statinen, den Fibraten, Nikotinsäure, den Ionenaustauschharzen, den Inhibitoren der Absorption von Cholesterol, den mehrfach ungesättigten Omega-3-Fettsäuren, Tiadenol, den Agonisten des Kernrezeptors FXR (Farnesoid X Rezeptor).

**18.** Zusammensetzung zu deren Verwendung nach Anspruch 9 oder 10, bei der Verwendung und/oder der Bekämpfung von Fettleibigkeit oder Übergewicht und/oder dem metabolischen Syndrom und/oder pathologischen Problemen der Arterienspannung.

## Claims

**1.** A composition comprising at least a mixture of molecules comprising at least:

- a single extract obtained from at least *Chrysanthellum indicum, Cynara scolymus, Vaccinium myrtillus, Olea europaea* and *Piper,* and/or
- a single extract obtained from at least *Chrysanthellum indicum, Cynara scolymus, Vaccinium myrtillus* and *Olea europaea* and of synthetic piperine,

said mixture of molecules comprising:

- at least one molecule chosen from apigenin-7-O-glucoronide, chrysanthelline A, chrysanthelline B, caffeic acid, luteolin, maritimetin, eriodictyol, isookanine, apigenin, luteolin-7-O-glucoside, maritimein, marein, eriodictyol-7-O-glucoside, flavomarein, apigenin-8-C-α-L-arabinoside-6-C-β-D-glucoside (shaftoside), apigenin-6,8-C-di-β-D-glucopyranoside (vicenin-2), and
- at least one molecule chosen from dicaffeoylquinic acid, sulfomonocaffeoylquinic acid, luteolin, luteolin-7-O-glucoside, luteolin-7-O-glucoronide, apigenin-7-O-glucoside, cynaropicrin, and
- at least one molecule chosen from monocaffeoylquinic acid, delphinidin-3-galactoside, delphinidin-3-gluycoside, cyanidin-3-galactoside, delphinidin-3-arabinoside, cyanidin-3-glucoside, petunidin-3-galactoside, cyanidin-3-arabinoside, petunidin-3-glucoside, peonidin-3-galactoside, petunidin-3-arabinoside, peonidin-3-glucoside, malvidin-3-galactoside, malvidin-3-glucoside, malvidin-3-arabinoside, and
- at least one molecule chosen from oleuropein and hydroxytyrosol, and
- at least piperine.

**2.** The composition according to either of the preceding claims, **characterised in that** the *Piper* is chosen from *Piper nigrum, Piper aduncum* and/or *Piper longum.*

**3.** The composition according to any of the preceding claims, **characterised in that** the single extract(s) are obtained from :

- *Chrysanthellum indicum* whole plant and/or aerial parts,
- the complete plant and/or from leaves of *Cynara scolymus,*
- the complete plant and/or from fruits of *Vaccinum myrtillus.*

**4.** The composition according to any of the preceding claims, **characterised in that** it has at least one of the following characteristics:

- it comprises a quantity of extract of *Chrysanthellum indicum* allowing the administration of at least 0.00001 g of extract of *Chrysanthellum indicum* per kg of body weight of the person to whom the composition is administered and per day, and/or
- it comprises a quantity of extract of *Cynara scolymus* allowing the administration of at least 0.00001 g of extract of *Cynara scolymus* per kg of body weight of the person to whom the composition is administered and per day, and/or
- it comprises a quantity of extract of *Vaccinium myrtillus* allowing the administration of at least 0.00001 g of extract of *Vaccinium myrtillus* per kg of body weight of the person to whom the composition is administered and per day, and/or
- it comprises a quantity of piperine allowing the administration of at least 0.001 mg of piperine per kg of body weight of the person to whom the composition is administered and per day.
- it comprises a quantity of unique extract allowing the administration of at least 0.00001 g of extract of unique extract per kg of body weight of the person to whom the composition is administered and per day.

**5.** A composition according to any of the preceding claims, **characterised in that** said mixture comprises at least dicaffeoylquinic acid, apigenin-7-O-glucuronide, monocaffeoylquinic acid, piperine and oleuropein.

**6.** The composition according to any of the preceding claims, **characterised in that** it also comprises at least one supplementary element added in addition to the mixture of molecules, said supplementary element being chosen from:

- the following vitamins: B1, B2, B3, B5, B6, B8, B9, B12, C, A, D, E, K1 and K2;
- the following compounds: obeticholic acid, corosolic acid, polyunsaturated fatty acids in the omega-6 and/or omega-3 family, orotic acid, pangamic acid, para-amino-benzoic acid, amygdalin, beta-glucans, carnitine, dimethylglycine, imeglimin, isoflavones, L-arginine, oxytocin, pectin, pyridoxamine, resveratrol, viniferine, L-citrulline;
- the following oligoelements and minerals: arsenic, boron, calcium, copper, iron, fluorine, iodine, lithium, manganese, magnesium, molybdenum, nickel, phosphorus, selenium, vanadium and zinc;
- the following microconstituents of a non-essential nature: conjugated linolenic acid, lipoic acid, carotenoids, carnitine, choline, coenzyme Q10, plant sterols, polyphenols in the tannin and lignan family, and taurine;
- fructo-oligosaccharides, galacto-oligosaccharides;
- lactic ferments;
- yeasts;
- fungi;
- products derived from insects compatible with the food and pharmaceutical sectors;
- marijuana and hashish;
- enrobing agents;
- flavourings;
- acidifiers;
- anticoagulants;
- thickeners;
- stabilisers;
- emulsifiers;
- fillers;
- excipients.

**7.** The composition according to any of the preceding claims, **characterised in that** it is in powder, gel or emulsion form, or in liquid form.

**8.** The composition according to any of the preceding claims, **characterised in that** it is in the form of tablets, pills, capsules, sticks, sachets, ampoules or droppers, or in injectible form.

**9.** The composition according to any of the preceding claims, for use as a medication.

**10.** The composition according to any of claims 1 to 8, for use as a medication or a nutrition product in the prevention of and/or combating against pathological ailments of the glucide and/or lipid metabolism in humans or animals.

**11.** The composition for use according to claim 9 or 10, in the prevention of and/or combating against type 1 and 2

diabetes and/or non-alcoholic fatty liver diseases and/or cardiovascular pathologies and/or pathologies related to insulin resistance.

**12.** The composition for use according to claim 11, **characterised in that** the non-alcoholic fatty liver disease is non-alcoholic hepatic steatosis.

**13.** The composition for use according to claim 11, **characterised in that** the cardiovascular pathologies are coronary heart diseases, cerebrovascular ailments, peripheral arteriopathies and deep vein thromboses.

**14.** The composition for use according to claim 11, **characterised in that** the pathology related to insulin resistance is Alzheimer's disease.

**15.** The composition for use according to any of claims 11 to 14, **characterised in that** it is used in combination with at least one antidiabetic therapeutic agent chosen from biguanides including metformin, dipeptidyl peptidase-IV (DPP-IV) inhibiters, glucagon-like peptide-1 (GLP-1) analogues, thiazolidinediones (TZDs), sulfonylureas, fast and slow insulins, sodium glucose co-transporter-2 (SGLT2) inhibiters, glycosidase inhibiters (acarbose, miglitol, voglibose, peptides containing the alanine-proline or proline-alanine sequence), molecules in the fibranor family such as elafibranor, or molecules targeting the nuclear receptors and in particular the ROR receptors (α, β, γ) and Rev-Erb receptors (α, β).

**16.** The composition for use according to claim 9 or 10, in the prevention of and/or combating against dyslipidaemia.

**17.** The composition for use according to the preceding claim, **characterised in that** it is used in combination with a hypolipidaemic therapeutic agent chosen from: statins, fibrates, nicotinic acid, ion exchange resins, cholesterol absorption inhibiters, polyunsaturated omega-3 fatty acids, tiadenol, and agonists of the FXR nuclear receptor (Farnesoid X receptor).

**18.** The composition for use according to claim 9 or 10, in the prevention of and/or combating against obesity and excessive weight and/or metabolic syndrome and/or pathological blood-pressure problems.

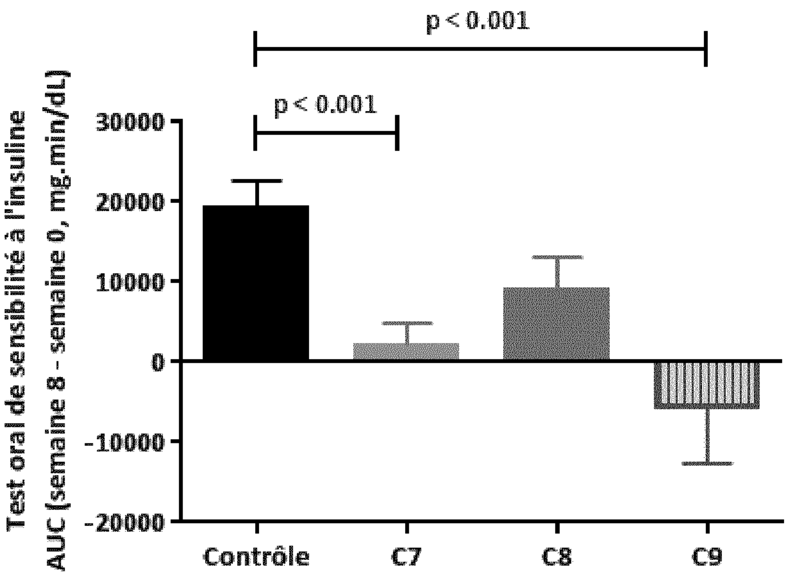


Figure 1

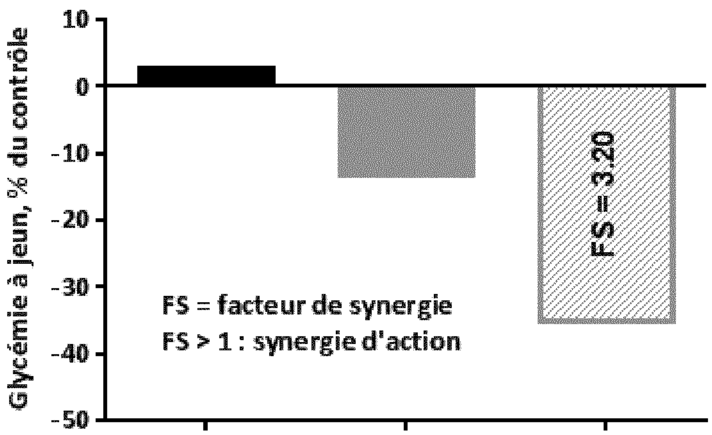


Figure 2

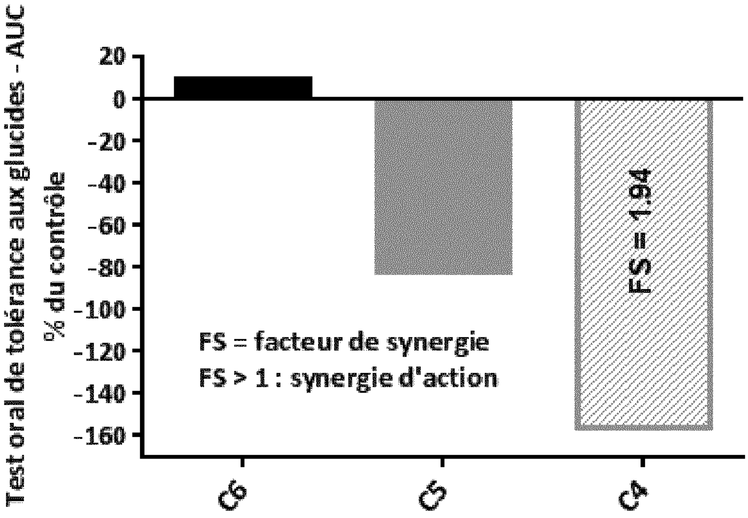


Figure 3

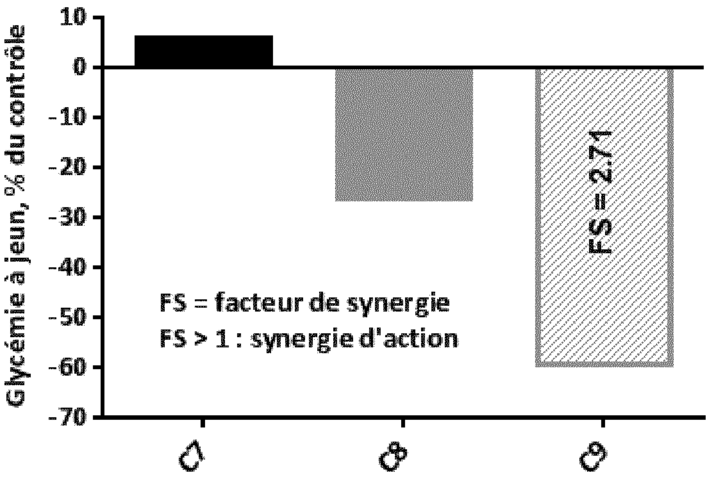


Figure 4

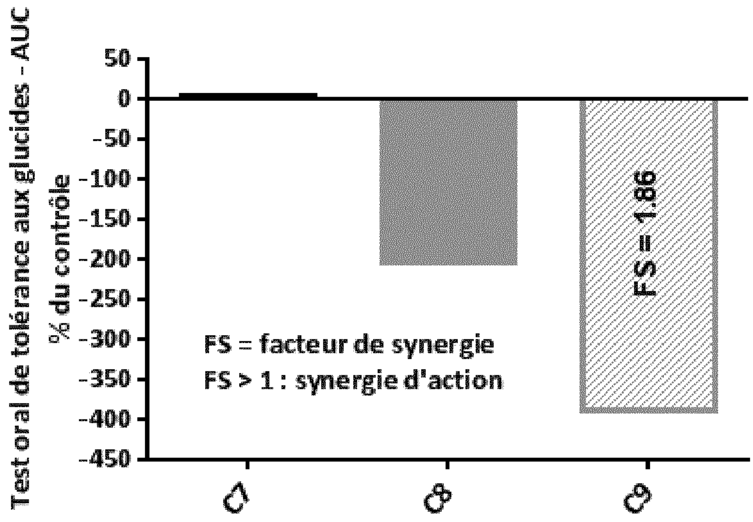


Figure 5

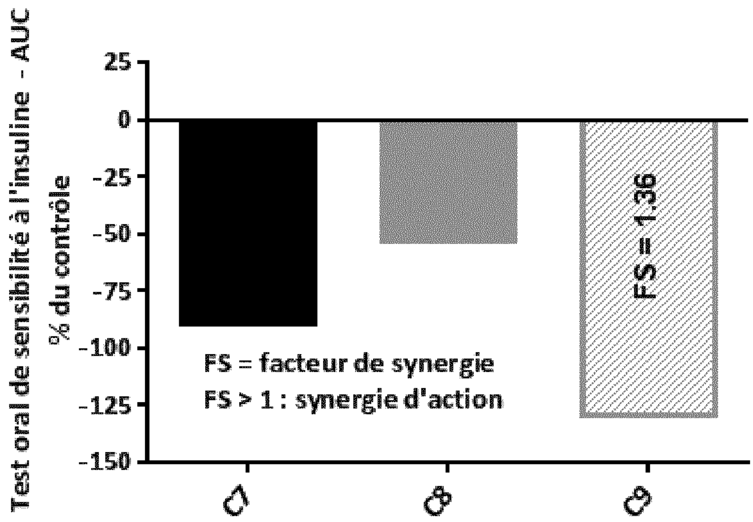


Figure 6

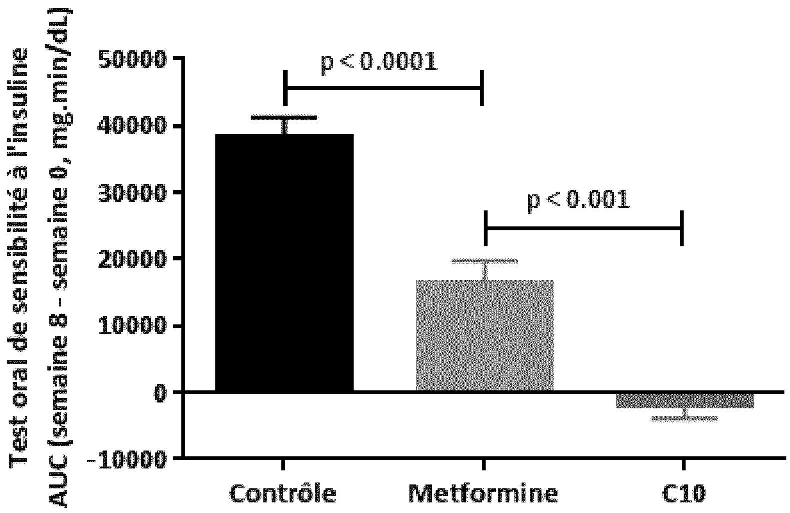


Figure 7

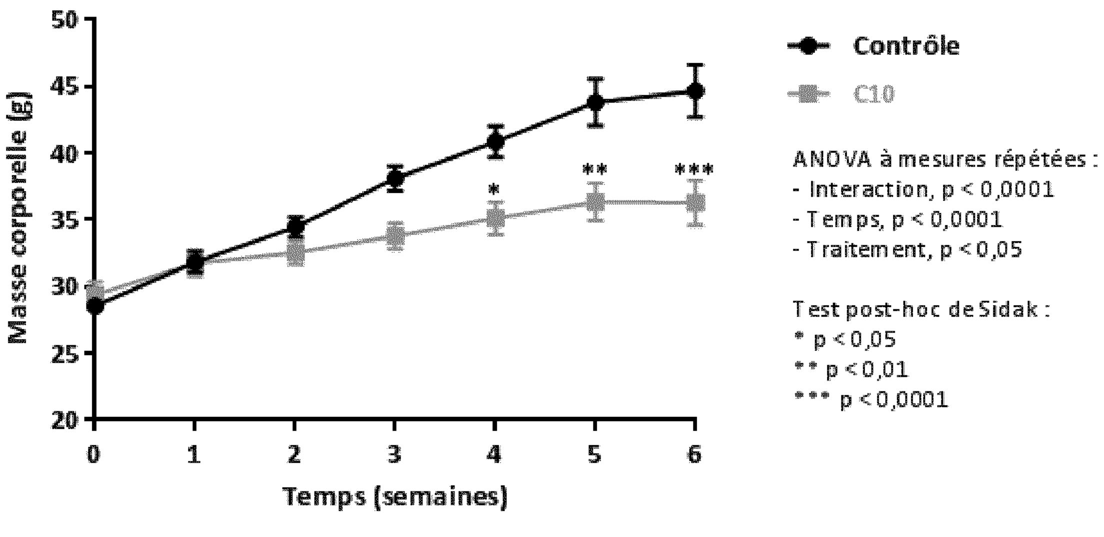


Figure 8

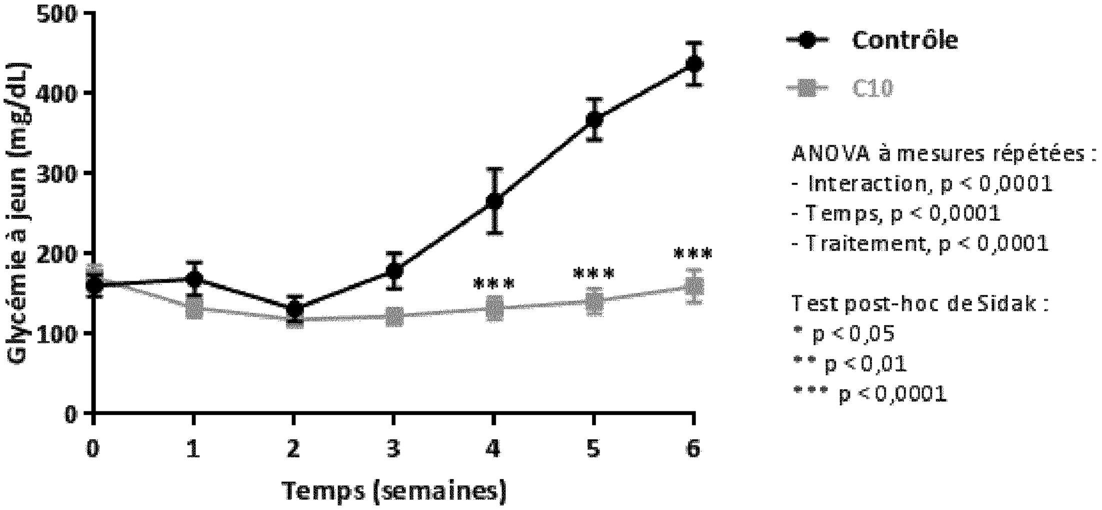


Figure 9

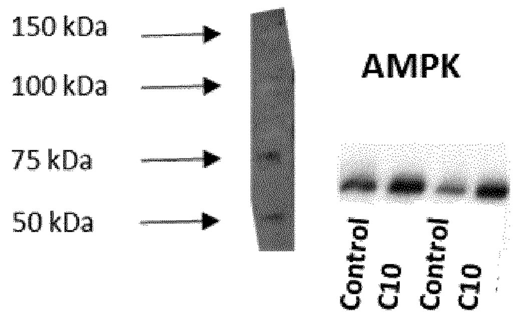


Figure 10

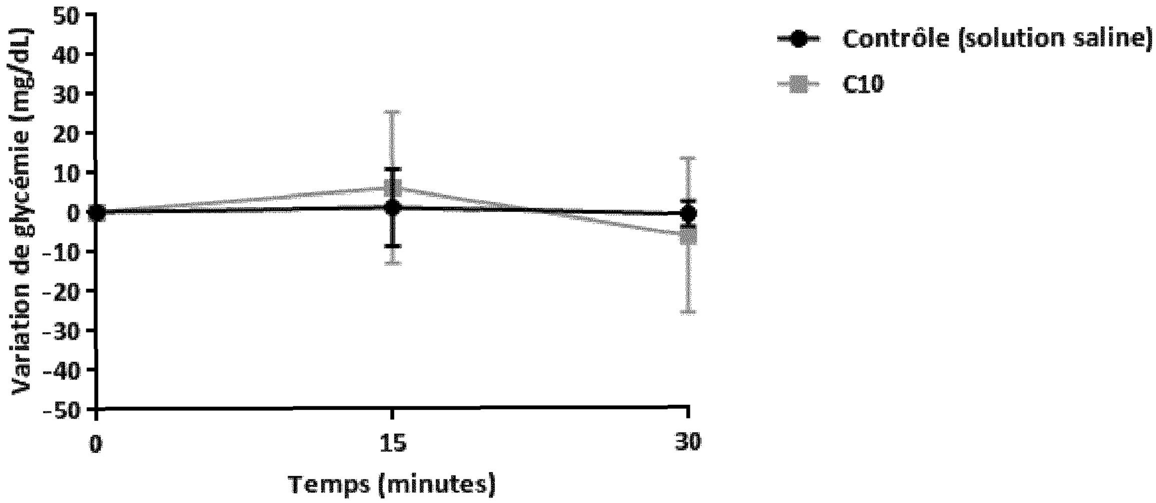


Figure 11

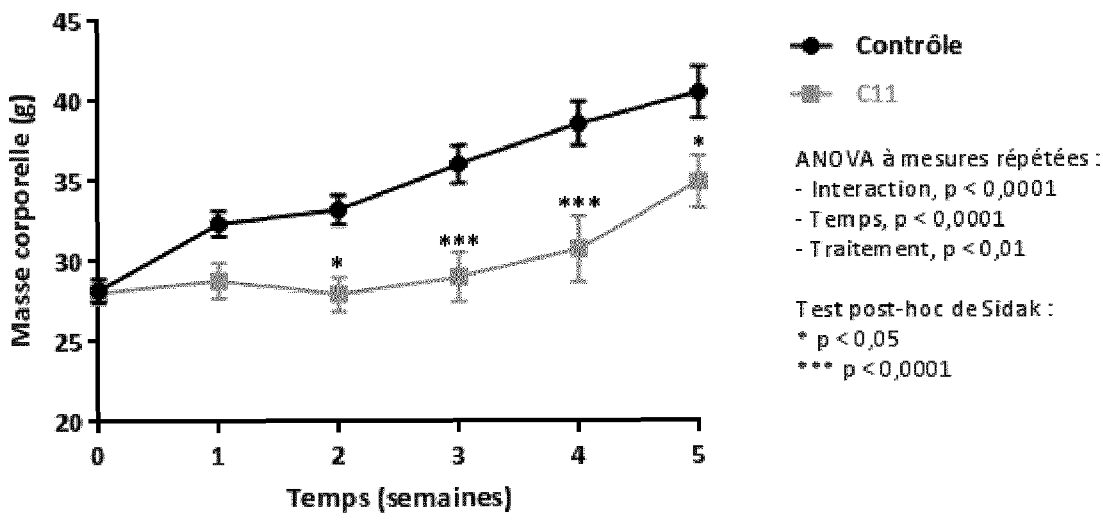


Figure 12

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- EP 03812880 A


### Littérature non-brevet citée dans la description


- *Ismail-Beigi F N Engl J Med,* 2012, vol. 366, 1319-27
- *Prescrire Int,* 2015, vol. 24, 130-5
- **DENKE MA.** *J Manag Care Pharm,* 2003, vol. 9, 17-9
- **BEDSE G et al.** *Front Neurosci,* 2015, vol. 9, 204
- **AILEEN JF ; KING BR.** *J Pharmacol,* 2012, vol. 166 (3), 877-894
- **SANCHES SC et al.** *Biomed Res Int,* 2015
- **SAMUEL VT et al.** *Cell,* 2012, vol. 148, 852-71
- **COLBY SR.** Calculation of the synergistic and antagonitic responses of herbicide combinations. *Weeds,* 1967, vol. 15, 20-22
- **FERRANNINI E et al.** *Eur Heart J,* 10 Juin 2015
- **HANF R et al.** *Diab Vasc Dis Res,* 2014, vol. 11, 440-7
- **LEAHY JL et al.** *J Clin Invest,* 1985, vol. 77, 908-915
- **COUGHLAN KA et al.** *Diabetes Metab Syndr Obes,* 2014, vol. 7, 241-53
- **KIM JS et al.** *Biosci Biotechnol Biochem,* 2000, vol. 64, 2458-61
- **VON GELDERN TW et al.** *Drug Development Research,* 2006, vol. 67, 627-42
- **GREEN BD et al.** *Diabetes Vasc Dis Res,* 2006, vol. 3, 159-65